# EUROPEAN PATENT APPLICATION

(11) **EP 1 199 364 A2**
(43) Date of publication of application: **24.04.2002**
(21) Application number: 01660180.9
(22) Date of filing: 25.09.2001
(51) Int. Cl.: C12N 15/53, C12N 15/54, C12N 15/60, C12N 15/62, C12N 15/63, C12N 9/04, C12N 9/10, C12N 9/12, C12N 9/88, C12N 1/15, C12N 1/19, C12P 19/32, C12P 21/00, G01N 33/53

(54) **Use of recombinant enzymes for preparing GDP-L-fucose and fucosylated glycans**

(30) Priority: 26.09.2000 FI 20002114
(71) Applicant: MediCel oy, 00290 Helsinki (FI)
(72) Inventor: Renkonen, Risto, 02160 Espoo (FI); Mattila, Pirkko, 02320 Espoo (FI); Hirvas, Laura, 00870 Helsinki (FI); Hortling, Solveig, 00250 Helsinki (FI); Kallioinen, Tuula, 01390 Vantaa (FI); Kauranen, Sirkka-Liisa, 02210 Espoo (FI); Järvinen, Nina, 09430 Saukkola (FI); Mäki, Minna, 00250 Helsinki (FI); Niittymäki, Jaana, 02730 Espoo (FI); Räbinä, Jarkko, 00530 Helsinki (FI)
(74) Representative: Boije-Backman, Solveig Magdalena

(57) **Abstract**

Use of recombinant enzymes for the preparation of GDP-L-fucose and fucosylated glycans is disclosed. GDP-L-fucose functions as a fucose donor in the biosynthetic route leading to the fucosylated glycans, which have therapeutic utility. A process for preparing GDP-L-fucose and fucosylated glycans, and means useful in the process are provided. Said means include enzymes, chimeric enzymes, DNA sequences, genes, vectors and host cells. An assay for the determination of GDP-fucose and fucosyl-transferase, and a test kit therefore are also provided.

SEQUENCE LISTING

<110> Medicel Oy

<120> Use of Recombinant Enzymes for Preparing GDP-L-fucose
      and fucosylated glycans

<130> sequences 1-8

<140>
<141>

<160> 8

<170> PatentIn Ver. 2.1

<210> 1
<211> 1146
<212> DNA
<213> Helicobacter pylori

<400> 1
atgaaagaaa aaatcgcttt aatcaccggg gttaccgggc aagacgggag ctatctggct 60
gaatacttgc tgaatttggg ttatgaagtg catgggttaa aaaggcgctc ttctagcatc 120
aacacttcta ggatcgatca tttgtatgaa gatttgcaca gcgaacacaa aaggcgtttt 180
ttcttgcact atggggatat gaccgatagc tctaacctca tccatttgat cgctaccact 240
aagcccacag agatttataa tttagccgcg caaagccatg tgaaagtctc ttttgaaacc 300
ccagaataca ccgctaacgc tgatggtatt ggcacgctaa ggattttaga ggccatgcgg 360
attttgggct tagaaaagaa aacgcgcttt tatcaagcta gcacgagcga attgtatggc 420
gaagtcttag aaaccccaca aaatgaaaac acccccttta acccacgaag cccctatgcg 480
gtcgctaaaa tgtatgcctt ttacatcact aaaaattaca gagaggctta taatttgttt 540
gcggttaatg gcatcctttt taaccatgag agcagggtaa ggggcgaaac ttttgtaacc 600
cgtaaaatca cacgagccgc tagcgcgata gcgtataact taacggattg cttgtattta 660
gggaatttgg acgctaaaag agactggggg catgccaaag attatgtgaa aatgatgcat 720
ttgatgctcc aagcacccac cccacaagat tatgtaatcg ctacagggaa gaccacgagc 780
gtgcgcgatt ttgtgaaaat gagctttgaa tttatcggca ttgatctaga atttcaaaat 840
acaggattaa aagaaatcgg tttgattaaa agcgttgatg aaaaaagagc gaacgcttta 900
caattaaact taagccattt aaaaacgggc aaaatcgtgg tgcgtataga cgagcactat 960
ttcaggccta ctgaagtgga tttgctctta ggcgatccca ctggggctga gaaagagctg 1020
ggctgggtta gggaatacga tttaaaagag ttggttaagg acatgttaga atacgattta 1080
aaagaatgcc agaaaaacct ttacttgcaa gatgggggct atactttaag gaatttttat 1140
gaatga                                                            1146


<210> 2
<211> 933
<212> DNA
<213> Helicobacter pylori

<400> 2
atgaatgaga tcattttaat caccggcgct tatggcatgg tggggcagaa cacggcgttg 60
tattttaaaa aaaacaagcc tgatgttacc ttactcaccc ctaaaaagag cgaattgtat 120
ttgttggata aagacaacgt tcaagcctat ttgaaagaat acaagcctac aggcattatc 180
cattgtgccg ggagagtggg gggcattgtg gcaaacatga acgatctttc aacttacatg 240
gttgagaatt tactcatggg tttgtatctt ttttctagcg ctttagattt gggcgtgaaa 300
aaagccatta atctagcgag ctcttgcgct tatcctaaat acgcccctaa ccctttaaaa 360
gagagcgatt tattgaacgg ctctttagaa ccaacgaatg aaggctacgc tttagccaaa 420
ctctctgtga tgaagtattg cgaatacgtg agcgctgaaa aaggcgtttt ttataaaact 480

ctagtgcctt gtaaccttta tggcgagttt gacaagtttg aagaaaagat agcgcacatg 540
ataccagggc ttattgctag gatgcacacc gctaaattaa aaaatgaaaa aaattttgcg 600
atgtggggcg atggcacggc cagaagagag tatctaaacg ctaaagattt agccagattc 660
atcgctctcg cttatgagaa tatcgctcaa atgcctagcg tgatgaatgt cggctctgga 720
gtggattaca gcattgaaga gtattacgaa aaagtcgctc aggttttaga ctataagggc 780
gtgtttgtga aagattcatc caaaccagtg ggcatgcaac aaaagcttat ggatatttcc 840
aaacaaaagg ctttaaaatg ggaattagaa atccctttag agcagggcat caaagaagct 900
tatgagtatt atttgaagct tttagaggtt tga 933

<210> 3
<211> 2388
<212> DNA
<213> Mouse

<400> 3
atggagcagt cagagggagt caattggact gtcattatcc tgacatgcca gtacaaggac 60
agtgtccagg tctttcagag agagctggag gtaaggcaga gacgggagca gattcctgcg 120
gggacgatgt tactggctgt ggaggatccc cagactcgag tcggcagcgg aggagccacc 180
ctcaacgcac tgctggtggc tgctgaacac ttgagtgccc gagctggctt cactgtggtc 240
acgtccgatg tcctgcactc tgcctggatc ctcatcttgc acatggccgg agacttcccc 300
ttcgatgact gtggcagggc cttcacttgc ctccctgtgg agaacccaca ggcccctgtg 360
gaggccttgg tatgcaacct ggactgcctg ttggatatca tgacccaccg gctgggtcca 420
ggttccccac caggtgtgtg ggtctgcagc accgacatgc ttctgtctgt tcctccaaac 480
cctgggatca gttgggatgg cttccgggga gccagagtga tcgcctttcc tgggagcctg 540
gcctatgcgt tgaaccacgg tgtctacctc actgactcac agggcttggt tttggacatt 600
tactaccagg gcactaaggc ggagatacaa cgttgtgtcg gacctgatgg gctggtacca 660
ttggtctccg ggtcgtcttt cttctctgtg gagactgctg agcacctcct agccacccat 720
gtgagcccac cgctggatgc ctgcacctat atgggcttgg actctggagc ccagcctgtg 780
cagctgtctc tgtttttcga catcctgctc tgcatggctc ggaatatgag cagggagaac 840
ttcctggctg ggcggccccc ggagttgggg caaggtgaca tggatgtagc aagttacctg 900
aagggagccc gggcccagct gtggagggag cttcgagatc agcccctcac aatggtgtat 960
gtccctgacg gcggctacag ctacatgacg actgatgcca ccgagttcct gcacagactc 1020
acgatgcctg gagtagctgt ggcacagatt gttcactccc aggtggagga gccacagctg 1080
ctagaggcta cgtgctcggt ggtcagctgc ctgctcgagg gccctgtgca cctggggcct 1140
cgaagtgtcc tgcagcactg tcacctgagg ggccccattc gcatcggcgc tggctgcttt 1200
gtgagtggtc tggatacagc ccactcggag gcactgcatg gcctggagct ccatgatgtc 1260
atcctgcagg gacaccatgt gcggctgcat ggctccctga gccgtgtatt tactcttgct 1320
ggccgtctgg acagctggga aaaaaagggg gcaggcatgt atctcaacat gtcctggaat 1380
gagttcttca agaagacagg cattcgagac tgggacctgt gggaccaaga tacacccccc 1440
tcagatcgag gcctcctcac tgcccgcctt ttccctgtgc tccaccccac gagggccctg 1500
gggccccagg atgtgctgtg gatgctgcac ccccgcaaac acagaggtga ggcccttcgg 1560
gcctggcgag cctcctggcg tctgtcctgg gagcagctgc aaccttgtgt ggaccgggct 1620
gccacactgg acttccgccg agatctgttc ttctgccagg ccttgcagaa ggcaaggcat 1680
gtgttagagg cgcggcagga cctctgccta cgtccactga tccgggccgc tgtcggggaa 1740
ggttgctttg ggcccctgct ggccacactt gacaaggttg cagctggggc agaagatcct 1800
ggcgtggcag cccgggctct ggcttgtgtg gccgatgtgc tgggctgcat ggcagagggt 1860
cgaggaggct tgcgcagtgg gccagctgcc aaccctgagt ggattcagcc tttctcatac 1920
ttggagtgtg gagacctgat gaggggtgtg gaggcgcttg cccaggagag agagaagtgg 1980
ctgaccaggc ctgccttgct ggttcgagct gcccgccatt acgagggggc cgagcagatc 2040
ctgatccgcc aggctgtgat gacagcccgg cacttcgtct ccacccagcc catggagctg 2100
cccgcacccg ggcagtgggt ggtgactgag tgcccagccc gtgtggattt ctctgggggc 2160
tggagtgaca caccgcccat tgcctatgag cttggtggag cagtgttggg cctggctgtg 2220
cgggtggatg gccgccggcc catcggggcc aaagcacgcc gcatcccgga gcctgagctc 2280
tggctggcag tgggacctcg gcaggatgag atgaccatga ggatagtgtg ccggagcctg 2340
gatgacctgc gggattactg ccagcctcat gccccagggc cttgctga 2388

<210> 4
<211> 2469
<212> DNA
<213> Human

<400> 4
atggagcagc cgaagggagt tgattggaca gtcatcatcc tgacctgcca gtacaaggac 60
agtgtccagg tctttcagag agaactggaa gtgcggcaga agcgggagca gatccctgct 120
gggacgctgt tactggccgt ggaggaccca gagaagcgtg tgggcagcgg aggagccacc 180
ctcaacgccc tgctggtggc tgctgaacac ctgagtgccc gggcaggctt cactgtggtc 240
acatccgatg tcctgcactc ggcctggatc ctcattctgc acatgggtcg agacttcccc 300
tttgatgact gtggcagggc tttcacctgc ctccccgtgg agaaccccga ggcccccgtg 360
gaagccttgg tctgcaacct ggactgcctg ctggacatca tgacctatcg gctgggcccg 420
ggctccccgc caggcgtgtg ggtctgcagc accgacatgc tgctgtctgt tcctgcaaat 480
cctggtatca gctgggacag cttccgggga gccagagtga tcgccctccc agggagcccg 540
gcctacgctc agaatcatgg cgtctaccta actgaccccc agggccttgt tttggacatt 600
tactaccagg gcactgaggc agagattcag cggtgtgtca ggcctgatgg gcgggtgcca 660
ctggtctctg ggttgtcttc cttctctgtg gagactgccg agcgcctcct agccacccac 720
gtgagcccgc ccctggatgc ctgcacctac ctaggcttgg actccggagc ccggcctgtc 780
cagctgtctc tgttttttga cattctccac tgcatggctg agaacgtgac cagggaggac 840
ttcctggtgg ggaggccccc agagttgggg caaggcgatg cagatgtagc gggttatctg 900
cagagcgccc gggcccagct gtggagggag cttcgcgatc agccccttac catggcctat 960
gtctccagcg gcagctacag ctacatgacc tcctcagcca gtgagttcct gctcagcctc 1020
acactccccg ggctcctggg ggcccagatt gtgcactccc aggtggagga gcagcagctt 1080
ctggcggccg ggagctctgt ggtcagctgc ctgctggagg gccctgtcca gctgggtcct 1140
gggagcgtcc tgcagcactg ccacctgcag ggccccattc acataggcgc tggctgcttg 1200
gtgactggcc tggatacagc ccactccaag gccctgcatg gccgggagct gcgtgacctt 1260
gtcctgcagg gacaccacac gcggctacac ggctccccgg gccacgcctt caccctcgtt 1320
ggccgtctgg acagctggga gagacagggg gcaggcacat atctcaacgt gccctggagt 1380
gaattcttca agaggacagg tgttcgagcc tgggacctgt gggaccctga gacgctgccc 1440
gcagagtact gccttcccag cgcccgcctc tttcctgtgc tccacccctc gagggagctg 1500
ggaccccagg acctgctgtg gatgctggac caccaggagg atgggggcga ggccctgcga 1560
gcctggcggg cctcctggcg cctgtcctgg gagcagctgc agccgtgcct ggatcgggct 1620
gccacgctgg cctctcgccg ggacctgttc ttccgccagg ccctgcataa ggcgcggcac 1680
gtgctggagg cccggcagga cctcagcctg cgcccgctga tctgggctgc tgtccgcgag 1740
ggctgccccg ggcccctgct ggccacgctg gaccaggttg cagctggggc aggagaccct 1800
ggtgtggcgg cacgggcact ggcctgtgtg gcggacgtcc tgggctgcat ggcagagggc 1860
cgtgggggct tgcggagcgg gccagctgcc aaccctgagt ggatgcggcc cttctcatac 1920
ctggagtgtg gagacctggc agcgggcgtg gaggcgcttg cccaggagag ggacaagtgg 1980
ctaagcaggc cagccttgct ggtgcgagcg gcccgccact atgagggggc tggtcagatc 2040
ctgatccgcc aggctgtgat gtcagcccag cactttgtct ccacagagca ggtggaactg 2100
ccgggacctg ggcagtgggt ggtggctgag tgcccggccc gtgtggattt ctctgggggc 2160
tggagtgaca cgccacccct tgcctatgag cttggcgggg ctgtgctggg cctggctgtg 2220
cgagtggacg gccgccggcc catcggagcc agggcacgcc gcatcccgga gcctgagctg 2280
tggctggcgg tggggcctcg gcaggatgag atgactgtga agatagtgtg ccggtgcctg 2340
gctgacctgc gggactactg ccagcctcat gccccaggtc aggcaccctg ggacctctgc 2400
aggtggggct ggccagctgg gggctcgggg cagaagctag agactgcagg ccagtggggt 2460
ctggcctga 2469

<210> 5
<211> 3540
<212> DNA
<213> Mouse

<400> 5
   ccctctagat gcatgctcga gcggccgcca gtgtgatgga tatctgcaga attcgccctt 60
   aagtcggcca tggagactct ccgcgaagcc accctgcgga aactgcgcag attttctgag 120
   ctgagaggca aacccgtggc agctggagaa ttctgggatg tggttgcaat aacagcagct 180
   gatgaaaagc aggagctcgc ttacaagcaa cagttgtccg agaagctgaa aaaaaaggaa 240
   ttgcctcttg gagttcaata ccatgttttt ccagatcctg ctgggaccaa aattggaaat 300
   ggaggatcga cactttgttc ccttcagtgt ttggaaagcc tctgtggaga caaatggaat 360
   tctctgaagg tcctgctaat ccactctggt ggctacagcc aacgccttcc caatgcgagt 420
   gctttaggaa agatcttcac tgccttacca cttggtgaac ccttctatca gatgttggag 480
   ttaaaactag ccatgtacgt ggatttcccc tcaaacatga ggcctggagt cttggtcacc 540
   tgtgcagata acatcgaact ctacagtgtt ggggacagtg agtacattgc ctttgaacag 600
   cctggcttta ctgccttagc ccatccgtct agtctgggtg ttgccactac tcatggagta 660
   tttgtcttgc actctgacag ttccctacaa catggtgacc ttaagttaag acaatgctac 720
   aattccctcc acaaccccac cattgaaaac atgccccacc ttatttctgt gcaaagacaa 780
   ggaagctttg ctcaacagga cttgtctgga ggtgacactg actgtcctcc attgcacact 840
   gagtatgtct acacagatag cctgttttac atggatcgca aatcagccaa aaagttactt 900
   gattctatta aaagtgaagg cccactgaac tgtgaaatag atgccaatgg agactttatt 960
   caggcactgg ggcctggagc aactgcagag tacaccagga acacatctca tgtcactaaa 1020
   gaagagtccc agttgttgga catgaggcag aaaatattcc acctcctcaa gggaacacca 1080
   ctgaatgttg ttgttcttaa taactccaga ttttatcaca ttggaacact gcaagagtat 1140
   ctgcttcatt tcacctctga tagtgcatta aagacgagag ctgggcttac aatccatagc 1200
   tttcaagtgt ctctccaagt gttcctgagc gctccagtgg aacagcctgt gtcattcaca 1260
   gtatactggg attcaggatg ctgtgtggcc cctggctcag tggtagagta ttctagattg 1320
   gggcctgagg tgtccatcgg ggaaaactgc attatcagca gttctgtcat agcaaaaact 1380
   gttgtgccag catattcttt tttgtgttct ttaagtgtga agataaatgg acacttaaaa 1440
   tattctacta tggtgtttgg catgcaagac aacttgaaga acagtgttaa aacactggaa 1500
   gacataaagg cacttcagtt ctttggagtc tgttttctgt cttgtttaga catttggaat 1560
   cttaaagcta cagagaaact attctctgga aataagatga atctgagcct gtggactgca 1620
   cgcattttcc ctgtctgttc atctctgagt gagtcggcta cagcatccct tgggatgtta 1680
   agcgctgtaa ggaaccattc accattcaac ctaagtgact ttaacctttt gtccatccag 1740
   gaaatgcttg tctacaaaga tgtacaagac atgctagctt atagggaaca catttttcta 1800
   gaaattagtt caaataaaaa tcaatctgat ttagagaaat cttaaattga tattttggcc 1860
   ataaacaaaa ttgcaaatac aggcattttc tatagacctc tgacattttt gtttgtttta 1920
   ataaagtaat ataataaaaa ttatgttaat ataactgttg tagcttggta atgagaatgg 1980
   tacaactgac cacttctgct agaagtacgt tccaggacta gagtcaggaa aggtcggctg 2040
   ttttagatgt ttacaccatc ttacaattgt gctctttggt aaagatccat ttatgggcac 2100
   tgtttcattc acaaaataaa tattctggtt tatagggatg atttcttaac ataacatatc 2160
   ttttaagctt ttctatctct tttggaaatt tggaccaata aaattctagg tgatattgga 2220
   ggatggtatt gctcaacttc tcatagtgag acaccccgta caaaacatgg ttttaaatct 2280
   tagaagaaat gtcatatttg agggttcttt gaggcttgtc aaggtttaat ttaaggggga 2340
   aaaattgttt acccggaagg aaggaatggc cccaattggc ttggggcacc aaaaagggtt 2400
   aaaaaaactg tttccaaaaa ttggggtggg acccttttgg gggcccaaac aatccttttg 2460
   gggggaacct aaggccattg gaaaaccaaa antttccatt attgtttcaa acaggaggga 2520
   attcccagtt agaagcacca aaaaatattt taaattgggg tcacacagga atgggacttg 2580
   attgaaggat accaggctcc ggaggtgaaa acctccggtt tagaaaaagc catgggtctt 2640
   ttggaactaa agttaaagaa gcttagaccc ccggggtggt tctggtagcc cacctccttt 2700
   agtctatgaa gtgctaaaaa tgaggttgtg cctccatggt cttgccaaat gatataaaag 2760
   atgtatggat gattttgttc ttatacacta gaacatgtgt tgccatatct tataaactat 2820
   gtctacttga tatattacac tggtagctat gtacacacag aactcagttg tctgctcagg 2880
   aggtggtagg gatagttgag agccagtact cactcactat ggaccttact taatcctctc 2940
   ctagttaatc cttctccaaa tctcttaact tgacagtgga catttgcctt gcatcattgg 3000
   tggtagtgat gctgtgaaca aacaataggc ccaaagagag gaaattcaaa taggcaatct 3060
   gaagaactac tcaaatcata aacaactgca gggaaatgaa atgggggaat tcctggttat 3120
   gcgtacctat tatgaaataa acacattagt ggaatgtcct taggttgaac tgtaatagag 3180
   ttaaatttta tcatacttgt gtttaaaata ccttaagtac attgtaatat ctgctgtggc 3240
   aactttaatt ctgtgtaagt tttcataaaa atatatgata aacaagatat ctgtcaaaac 3300
   tcctttatat tatttatata agaatatttg cctttttgag gtactagata ataaagcaaa 3360
   gaatgtacga tactatatga caattattgg taaagttaca gagaattcaa tggatgttaa 3420
   atgttattaa atactcaaga ctaaagtcct atcaacgatg agaattatga tttcatgttc 3480
   caagaaaaaa atatcattaa taaagaatac catcacttcc ttgtaaaaaa aaataaaaaa 3540

<210> 6
<211> 3461
<212> DNA
<213> Rat

<400> 6
ctcactatag ggctcgagcg gccgcccggg caggtgtggg ctcccggaag tcggccatgg 60
agactctccg ggaagccacc ctgcggaaac tgcgcagatt ttcggagctg agaggcaaac 120
ctgtggcagc tggagaattc tgggatgtgg ttgcgataac agcagccgat gaaaagcagg 180
agctcgctta caagcagcag ttgtcagaaa agctgagaag aaaggaattg cctcttggag 240
ttcaatacca tgtttttcct gatcctgctg ggaccaaaat tggaaatgga ggatcgacac 300
tttgttccct tcagtgccta aaaagcctct atggagatga atggaattct ttcaaggtcc 360
tgttaattca ctccggtggc tacagtcaac gccttcccaa tgcaagtgct ttaggaaaga 420
tcttcacagc cttaccactt ggtgaaccca tctatcagat gttggagtta aaactagcca 480
tgtacgtgga tttcccctca cacatgaagc ctggagtctt ggtcacctgt gcagatgaca 540
ttgaactgta cagtgttggg gactgtcagt acattgcctt tgaccagcct ggctttactg 600
ccttagccca tccttccagt ctggctgtag gcaccacaca cggagtattt gtcttgcact 660
ctgccagttc cttacaacat ggtgaccttc agtacagaca atgccaccgt ttcctccaca 720
agcccaccat tgaaaacatg catcagttta atgctgtgca aagacaagga agctttgctc 780
aacaggactt ccctggaggt gacaccgcgt gtcttccatt gcacactgag tatgtctaca 840
cagatagcct gttttacatg gaccacaaat cggccaaaaa gttacttgat ttctataaaa 900
atgtaaacca actgaactgt gaaatagatg cctatggtga ctttctgcag gcactggggc 960
ctggagcaac tgcagagtat accaggaaca catcacatgt cactaaagaa gactcccagt 1020
tgttggacat gaggcagaaa atattccacc tcctcaaggg gacaccactg aatgttgttg 1080
ttcttaataa ctccagattt tatcacattg gaacaacaca agaatatctg cttcatttca 1140
cgtctgatag tacgttaagg tcaagagcta ggcttacagt ccatagcttt caagtgtctc 1200
tccaagtatc cctgaatcct ccaatgaaac agcctgtatc attcacagta tactgggatt 1260
caggatgctg tgtggcacct ggctcagttg tagagtattc tagactgggg cctgaggtgt 1320
ccattgggga aaactgcatt gtcagcagct ctgtcctagc aaacactgct gtgccggcat 1380
attcttttgt gtgttctcta agtgtgagga caaatggact cttggaatat tctaccatgg 1440
tgtttagtgt gcaggacaac ttgaaaggca gtgttaaaac cctggaagat ataaaggcac 1500
ttcagttctt tggagtctgt ttcttgtctt gtttagacat ctggaacctt aaagctacag 1560
agaaactgtt ctctggaagt aagaggaacc tgagcctgtg gactgcacgg attttccctg 1620
tctgtccttc tctgagtgag tcagttacag catcccttgg gatgttaagt gctgtaagga 1680
gccattcacc attcagccta agcaacttta agctgatgtc catccaggaa atgcttgtct 1740
acaaagatgt acaagacatg ctagcttata gggagcaaat ttttctagaa attaattcaa 1800
ataaaaaaca atctgattta gagaaatctt aaattgaaca tattttgtct gtaaacaaaa 1860
ttgaaaatgc agatattttt ctatagacct gactttttgt ttagtaaatc aatataataa 1920
aagttatatt aataaaaagc gttgtagctt ggtaatgaat aaggtatggc tgagcacttc 1980
agctggaagg acattctggg actaaagtca ggaaagcaat gctgtttcag atgtttgcat 2040
catcttaatt ttgctttttg ggtgaaggtc catttacggg cactggtttg gttcacagaa 2100
taaatactcc tgactatggg atgattttct aaacatcctt tacagttttc ctatcttctt 2160
tggatttggc caataaagtc taggggatat tgatgattat attaccttac tctccaagtg 2220
agacaccatc tacaatacat tggtagaaaa ttcctaaaag aaattcattt ttgagggttt 2280
cttcaaaatt tgtcaagttt aagggacgaa gctttacttg catgtatgtt tgggcaccgc 2340
atgctgctct gccaaaagct aaacagctgt tctcacctgt ggggtgcctt tttgggatga 2400
accatttttg aggggtacct aagaccactg aaaataaaaa attcgcattg cgatttataa 2460
caggaaaatt acagttaaga agtagcacaa agtaatttta cagtttgggt caccacagcc 2520
ttaggaactg tttttaaagg atggcagcat caggaaggtt gagaaccact ggtctagaag 2580
aaggcattgg atctgttgga atcagtatag atggttacaa cctccatatt gtttagtctg 2640
caagtcaact ttagtcctat gaaagtgctt ataaaatgat gtttgtgcct cactgatctt 2700
gccaaaatga gattaaatgt acggtgtgtg ttattcttaa tacacttgaa catggggtgc 2760
catattttat aaactacatc actgatctat tgcactggaa aatgcataca cacagtatgc 2820
ggttgtctgc tcaggaggtg gtaggaatag ttgagagcca gtgctcactc agcgtggaca 2880
ttactttatc ctctcctaat taatccttca acaagtctct tgacaatgag catttgcctt 2940
acatcgtttt gtgtccatag tggtagtgat gctgtggaca agcaattggc ccaaagacag 3000
gaaattccaa taggaaatct gaggtaatac tcagatcata caggtagaat tcctggttaa 3060

aggcatctat tttgaaataa acacattagt ggaatgtctt taggttgaac ttgaatcgag 3120
gcgagactta tacttaagga aactgtaata tctactgtga catattactt atatgtaagt 3180
tttcataaaa atatatgata aacaagatat ctatcaaacc cctctttatt atttataatg 3240
aatatgtatt taaccttttg aggttctaga caataaagca aagtatacgt gatgatattt 3300
gacagtgatt gatttacagg gatctcaaag gatgttgaat gctattaaat gttcaaaact 3360
aaagtcccat caagtattag aattatgatt gcatgttcca aaaaatctat tattaataaa 3420
gaataccatc acttccttgc aaaaaaaaaa aaaaaaaaaa a 3461

<210> 7
<211> 1910
<212> DNA
<213> Rat

<400> 7
ggttgatgcc aaagattgaa ggggtagggt ggggcagaag tgggaaggtc cctggcttcc 60
tcaccttggt agatgaaaca aaagcatcag gctgagttga gcagtagctg tgatttcagg 120
gtgcctctgt tggagaggct gctgtgattt gaaaacctcc tttccctggg tgactaattc 180
cagaaagctc tggatgaatt gtattggtga gtgcctggcc ttgagaagtc ccagctgggg 240
acgatgggga ttttggggtg tccccgaacc taaggtgaca gggcctctcc tttttcattc 300
tgcttcaggg tgcaaccccg tctggaagct gcgggcctgg gggtgtctgg ctggaggtac 360
aacactcatg gtaatctggc tcttctggct gctgcgatca gttcctgggg gtgccccagc 420
gccccagccc acactcacca tccttatctg gcactggcct ttcaccaacc ggtcgccaga 480
gctgtctagc gacacctgca ctcgctatgg catggccagc tgccacctga gtgctaaccg 540
gagtctgcta gccagtgctg atgctgtggt cttccaccat cgtgagctgc agacccggca 600
ctctcgcctg cccctggacc agaggccgca cggacagcct tgggtctggg ccaccatgga 660
atcacccagt aatacccatg gtctccgtca cttccggggc atcttcaact gggtgctgag 720
ctatcggcgt gattcagata tctttgtacc ctatggtcgc ttagagccct tctctgggcc 780
tacgccccca ctaccagcca aaagcaggat ggctgcctgg gtggtcagca atttccagga 840
gcggcagcag cgggcaaagc tgtaccggca gctggcccct catctgaagg tggatgtgtt 900
cggtcgcgcc agtggacggc ccctgtgccc taactgtctg ctgcccactg tggcccggta 960
ccgcttctac ctgtcctttg agaactcaca gcaccgggac tacatcaccg agaagttctg 1020
gcgcaatgca ctggccgctg gcgctgtgcc tgtggtgctg ggacctcctc ggactaccta 1080
cgaggctttc gtgccaccag atgcctttat acacgtagat gacttcagct ctgcccgtga 1140
actagctgtc ttccttgtca gcatgaatga gagtcgctat cgtggcttct ttgcttggcg 1200
agaccggctc cgtgtgcggc tcctgaatga ctggagggag cgcttctgca ccatctgcgc 1260
ccgctaccct tacttgcccc gcagccaggt ctatgaagac ctggaaagct ggttccaagc 1320
ttgaactcct gctgctggga gaggctgtgt gcgtggaaga ctgatgatga aatggaaggg 1380
cttttgggtc accatgggac taaccctagg cttaggtcag tgaataggaa gtcaggatat 1440
gaggagaagg ctgggctgag aagccatatg gatgaggact ctggtgggct ttagagtagg 1500
gacccaggga aggagacaat taatgaggag cgtgtgggaa aagctagtca aacggggaaa 1560
gtggctgagg gtcctggact taccttgagt atgctcatgg ctcaaggctc aggtgaaaaa 1620
gggaggcagt gtctctggag ctgggaacat ccaaagctgg gatttgtggg gacaaacatg 1680
gtgcctgaac ctccacaatc aaagtgctta gcctcaggga tacaagtgtg tgttccagaa 1740
ctccacatgc aaaatgtatg ctgagcccag ggtttgtggg agaaggatgg tgtggatgat 1800
tctgggcttt tgacaccaca gttccctgag ggaaagaggc accactaata ataaaattgt 1860
tcacttgtaa tagagacgcc aaaaaaaaaa aaaaaaaaaa aaaaaaaagt 1910

<210> 8
<211> 1190
<212> DNA
<213> Helicobacter felis

<400> 8
atgcctttgt tgagagcacc aagctacccc cccccccccc ccacaaaaag cctttaaatt 60
tagacttaca atggtatcgc gatttaaatg attttaaggg gtggtttttt tatgacattt 120
tccagcacca atacaatgtt tcgtttgata aaaaggcgga ttgtgattgc ctccttggag 180

tgcaccatag aacactagaa gagttgtacc aagccttgac taatcccaaa aagcgtcttg 240
tgtttatggg agaaaatgag cgcattgatt ttaatgtcta cgactttgcc atgagttttg 300
atcatttgga gtttggagat cgctatttgc gcgtgcctct ttattaccag tctctccatt 360
ggttcttgca catcattact catgcgtcta atcctccttt tagactggat ggagcgcacg 420
agatgctaca ctctcctttg acactccatc taccccttaa ctccacatgc actaaaattt 480
cttttgctga caaataccct catttagatg ccctagctag agaacagaaa aatcctcttg 540
aacgagagtt tgctagtttt gtggcttcca actggggagc ccccatgcgc aataattttt 600
accagcaact caacgagtat cgccctgtgg ctgggggtgg gagagtgttt aacactatag 660
gaaaacccgt atctaataaa catgactttt tagcccaata taagttcaat ttgtgctttg 720
aaaactcttg tggcatgggt tataccactg agaaaattgt ggatgcttat tttgcccaca 780
ctattcctat ttattggggt aatccgttag tgcatttgga ttttaaccct aaaagttttg 840
tgaatgtcca tgattttgcc aacctagatg aggcgatcga ttttgtgcac tatttggaca 900
cccatgacaa tgcctattta gaaatgctcc atgctcaccc gcttagcatt gtggaaggca 960
aaccgaaatt ttgccatgat ttgagtttta agaaaatctt agactttttg gtcaatgcga 1020
tagaaagtcc gcataactat cacgagcagg tccgtgtact tagtaacagt gtgtacaaat 1080
atcggcatcc aagccgcgac accgtgctag aagcatgctc tggaagagaa catctgcaac 1140
tcctcctcaa aaagctccat aaaaaattat ggaagcgcaa gagaccctaa 1190

## Description

### Field of the Invention

The present invention relates to the use of recombinant enzymes for preparing GDP-L-fucose and fucosylated glycans. Fucosylated glycans are biologically active and have therapeutic utility, and GDP-L-fucose functions as a fucose donor in the biosynthetic route leading to the fucosylated glycans.

More precisely, the present invention is directed to a process for preparing GDP-L-fucose and fucosylated glycans, and to means useful in said process. Accordingly, the invention is also directed to DNA sequences and genes encoding useful enzymes and to the enzymes. Chimeric enzymes comprising several enzymes of the biosynthetic pathway are also provided. The invention further relates to vectors comprising the DNA sequences encoding the enzymes or chimeric enzymes, and to host cells comprising the vectors. Finally, the invention provides an assay useful in the process of the invention for the determination of GDP-fucose or fucosyltransferase, and a test kit therefore. Said assay may also be useful in the diagnosis of infection or inflammation.

### Background of the Invention

Fucosylated glycans are useful in the treatment of inflammatory responses. They can be used to block leukocyte traffic to sites of inflammation and thus reduce or otherwise ameliorate an undesired inflammatory response and other disease states characterized by a leukocyte infiltrate. They are also useful in blocking bacterial adherence to endothelium and thus they prevent and/or treat bacterial infections. A further use of the fucosylated glycans lies in the field of cancer treatment where metastasis of tumor cells can be inhibited by these glycans (US 5,965,544).

The migration of white blood cells from the blood to regions of pathogenic exposure in the body is called the inflammatory cascade. Cell adhesion events allow specific binding of a leukocyte to the endothelium of the vessel that is adjacent to the inflammatory insult; such adhesion events counteract the high vascular shear forces and high blood flow rates that tend to keep the leukocyte circulating, and help guide the leukocyte to the required site.

The current concept of leukocyte extravasation is based on the consecutive action of several adhesion molecules located on the surface of leukocytes and the endothelium. Lymphocyte extravasation is initiated by the interaction of members of the selectin family and their oligosaccharide-containing counterreceptors.

Selectins, also known as "lectin cell adhesion molecules" (LEC-CAMs), are classified into three groups: L-selectin is expressed on various leukocytes, and is constitutively expressed on lymphocytes, monocytes, neutrophils, and eosinophils. E-selectin is expressed on endothelium activated by inflammatory mediators. P-selectin is stored in alpha granules of platelets and Weibel-Palade bodies of endothelial cells and is also expressed on endothelium activated by inflammatory stimuli. All members of the selectin family appear to mediate cell adhesion through the recognition of carbohydrates.

All selectins bind to sialyl Lewis x (NeuNAcα2-3Galß1-4(Fucα1-3)GlcNAc) (sLe^{x} or sLex) and sialyl Lewis a (NeuNAcα2-3Galß1-3(Fucα1-4)GlcNAc) (sLe^{a} or sLea) as well as to related carbohydrate sequences. L-selectin-dependent recognition precedes normal lymphocyte extravasation into peripheral lymph nodes and into sites of inflammation, both of which are impaired in L-selectin deficient mice.

Several glycoproteins have been shown to act as counterreceptors for L-selectin. A common nominator for the cloned ligands GlyCAM-1, CD34, MAdCAM-1 and PSGL-1 is the mucin type protein core rich in O-linked glycan decorations which are crucial for selectin recognition. The glycosylation of GlyCAM-1 and PSGL-1 has been characterized in greater detail, among other saccharides these proteins have been shown to carry sulfated sLex and sLexLexLex epitopes, respectively .

High endothelial cells in peripheral lymph nodes express sialyl Lewis a and sialyl Lewis x (sLea and sLex) epitopes, which are parts of the L-selectin counterreceptor. The endothelial cells in several other locations are sLea and sLex negative, but inflammatory stimuli can induce previously negative endothelium to express these oligosaccharide structures *de novo.* It has been shown that cultured endothelial cells possess the machinery to generate at least sLex, since they have several functional α-2,3-sialyl- and α-1,3-fucosyltransferases, enzymes involved in generating sLex from (poly)lactosamines.

A number of studies have proposed that selectins are involved in a wide variety of acute and chronic inflammatory conditions in many tissues. Mono- and multivalent sLex glycans have been shown to inhibit L-selectin mediated lymphocyte binding *in vitro* and they also inhibit granulocyte extravasation in *in vivo* animal models of acute inflammation and reperfusion injury. Polylactosamines carrying single epitopes of sLexLex- and sLexLexLex-type appear to be recognized by E- and P-selectins with higher affinity than analogous oligosaccharides bearing single sLex-units. WO 97/12892 discloses synthetic multivalent sLex containing polylactosamines and their use to block lymphocyte binding to correspondent oligosaccharides on the endothelial surface.

Fucosylated, glycans such as sLex and/or Lex, have been shown to be crucial in selectin dependent extravasation of leukocytes and tumor cells as well as in bacterial and parasitic infections [Vestweber D, and Blanks JE: Mechanisms that regulate the function of the selectins and their ligands. Physiol. Rev. 1999, 79:181-213]. Fucosylation of glycans on glycoproteins and -lipids requires the enzymatic activity of relevant fucosyltransferases and GDP-L-fucose as the donor. Due to the biological importance of fucosylated glycans, a readily accessible source of GDP-L-fucose would be required. Currently GDP-L-fucose is still a relatively expensive nucleotide sugar and thus laboratories working in the field of fucosylation would benefit of more accessible sources of it.

There are two major strategies for the synthesis of GDP-L-fucose, the chemical and the enzymatical pathways. Various approaches have been used in the relatively complex chemical synthesis starting from L-fucose, the endproduct being GDP-L-fucose. Such syntheses are, however, very laborious, expensive and inconvenient for producing L-fucose on the large scale.

In eukaryotic cells GDP-L-fucose can be synthesized via two different pathways, either by the more prominent *de novo* pathway or by the minor salvage pathway. Procaryotes have only the *de novo* pathway. The predominant *de novo* route starts from GDP-D-mannose and the minor salvage pathway uses L-fucose as the starting material [Becker DJ, and Lowe JB: Leukocyte adhesion deficiency type II [Review]. Biochim. Biophys. Acta - Molecular Basis of Disease. 1999, 1455:193-204]

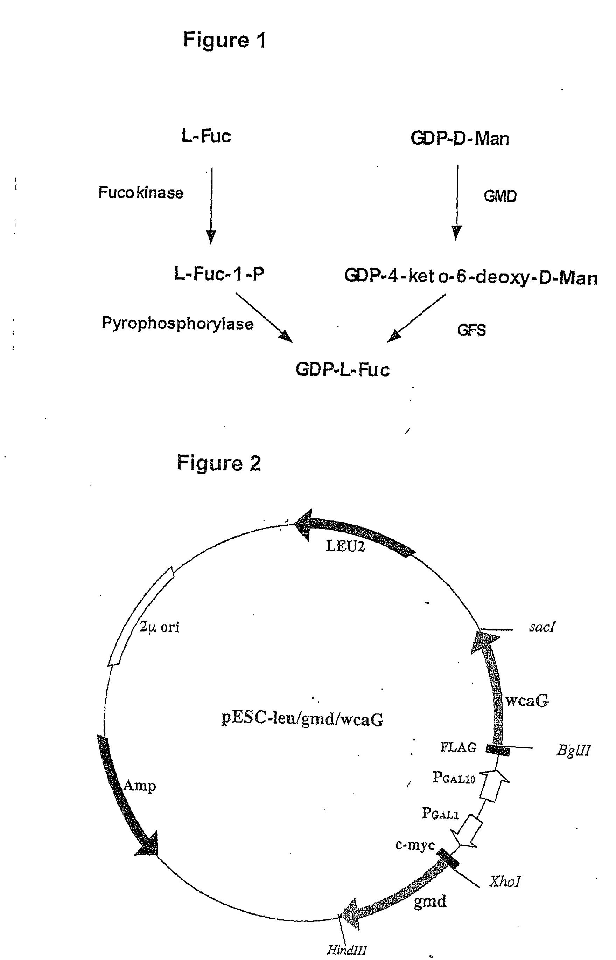

The first step of the *de novo* pathway starting from GDP-D-mannose is a dehydratation reaction catalyzed by a specific nucleotide-sugar dehydratase, GDP-mannose-4,6-dehydratase (GMD). This leads to the formation of an unstable GDP-4-keto-6-deoxy-D-mannose, which undergoes a subsequent 3,5 epimerization and to the formation of a GDP-4-keto-6-deoxy-D-galacatose, which then underogoes a NADPH-dependent reduction resulting in the formation of GDP-L-fucose (Fig. 1). These two last steps are catalyzed by a single, bifunctional enzyme GDP-4-keto-6-deoxy-D-mannose-3,5-epimerase/4-reductase (GFS), also called GDP-L-fucose synthetase. The genes coding for GMD and GFS activities for *de novo* pathway have been cloned from several bacteria, plants and mammals [Tonetti M, Sturla L, Bisso A, Benatti U, and De Flora A: Synthesis of GDP-L-fucose by the human FX protein. J. Biol. Chem. 1996, 271:27274-9].

The salvage pathways utilizes L-Fuc and converts it to GDP-Fuc via two enzymatic reactions (Fig 1). The two enzymatic steps are catalyzed by fuco-1-kinase (FK) and GDP-fucose-pyrophosphorylase (PP). FK converts L-Fucose into L-Fucose-1-P and PP converts the L-Fucose-1-P into GDP-L-fucose. The salvage pathway has been successfully performed with purified enzymes in a recycling one-pot approach, but not yet with recombinant enzymes [Ichikawa Y, Look GC, and Wong CH: Enzyme-catalyzed oligosaccharide synthesis. Analytical Biochemistry 1992, 202:215-38].

L-fucose is a crucial monosaccharide present in several biologically important glycans [Feizi T, and Galustian C: Novel oligosaccharide ligands and ligand-processing pathways for the selectins. TIBS 1999, 24:369-372]. In prokaryotes L-fucose is mainly present in polysaccharides of the cell wall and in animals L-fucose is a part in glycoconjugates, such as ABH-and Lewis antigens, either bound to the cell membrane or secreted into biological fluids. Fucosylation requires the action of fucosyltransferase, which catalyses the transfer of L-fucose from a nucleotide sugar, GDP-L-fucose, into the glycan acceptor.

The biosynthesis of GDP-L-fucose, which acts as a nucleotide-sugar donor for fucosylation, has received increased interest after a number of α-1,2-, α-1,3- and α-1,6-fucosyltransferase genes have been cloned, sequenced and expressed. Fucosyltransferases catalyze the transfer of fucose from a nucleotide sugar, GDP-L-fucose (GDP-Fuc), to a saccharide acceptor.

Some fucosyltransferases have been cloned from various sources and thus they have become available for synthetic purposes. However, GDP-Fuc is still a relatively expensive nucleotide sugar and therefore a more accessible source of it would be desirable. The availability and high costs limiting the large-scale oligosaccharide synthesis could be overcome by enzymatic synthesis of GDP-Fuc from cheaper carbohydrate resources.

One aim of the present invention is to increase the efficacy to synthesize GDP-L-Fuc. Another aim of the present is to facilitate the production of fucosylated glycans from GDP-L-Fuc. Fucosylated glycans have biologically useful properties, which can be utilized in glycobiology research and medicinal applications. The present invention thus provides processes and means for producing GDP-L-fucose and fucosylated glycans by recombinant gene technology.

Still another aim of the present invention is to provide an assay for the diagnosis of infection or inflammation.

### Summary of the Invention

One object of the present invention is to provide a process for preparing GDP-L-fucose and fucosylated glycans, wherein said compounds are prepared using one or more recombinant enzymes from their biosynthetic routes. Another object of the invention is the use of recombinant enzymes for preparing GDP-L-fucose and fucosylated glycans.

Further objects of the invention are isolated DNA sequences encoding *Helicobacter pylori* GDP-mannose-4,6-dehydratase (GMD), *Helicobacter pylori* GDP-4-keto-6-deoxy-D-mannose-3,5-epimerase/4-reductase (GFS), murine or human fuco-1-kinase (FK), rat or murine GDP-fucose-pyrophosphorylase (PP), or *Helicobacter felis* or rat α-1,3-fucosyltransferase.

Still further objects of the invention are the isolated genes comprising the DNA sequences and the enzymes encoded by the sequences.

The invention further provides a chimeric enzyme, which is a chimeric molecule of GDP-mannose-4,6-dehydratase (GMD) and GDP-4-keto-6-deoxy-D-mannose-3,5-epimerase/4-reductase (GFS), or a chimeric molecule of fuco-1-kinase (FK) and GDP-fucose-pyrophosphorylase (PP).

Additional objects of the invention are vectors comprising one or more of the DNA sequences set forth above and double vectors comprising a first DNA sequence encoding GDP-mannose-4,6-dehydratase (GMD) and a second DNA sequence encoding GDP-4-keto-6-deoxy-D-mannose-3,5-epimerase/4-reductase (GFS), or a first DNA sequence encoding fuco-1kinase (FK) and a second DNA sequence encoding GDP-fucose-pyrophosphorylase (PP). Host cells comprising the vectors are also provided.

Finally, the invention provides an assay for the determination of GDP-fucose, said assay comprising employing biotinylated carbohydratepolyacrylamide conjugates, streptavidin and time-resolved fluorometric detection, and test kits comprising reagents needed for performing the assays.

Preferred embodiments of the invention are set forth in the dependent claims.

### Brief Description of the Drawings

Figure 1 shows two biosynthetic routes for GDP-L-fucose.
Figure 2 shows the vector pESC-leu/gmd/wcaG.
Figure 3 shows the vector pESC-trp/mFK.
Figure 4 shows the vector pESC-trp/PP.

### Detailed Description of the Invention

Abbreviations used: AAL, *Aleuria aurantia* lectin; FucT, α-1,3-fucosyltransferase; GDP-Fuc or GDP-L-Fuc, GDP-L-fucose; GDP-Man or GDP-D-Man, GDP-D-mannose; GFS, GDP-4-keto-6-deoxy-D-mannose-3,5-epimerase/4-reductase (GFS) or GDP-L-fucose synthetase; GMD, GDP-D-mannose dehydratase; Lex, Lewis x [Galβ1-4(Fucα1-3)GlcNAc]; sLex, sialyl Lewis x [Neu5Acα2-3Galβ1-4(Fucα1-3)GlcNAc]; sLN, sialyllactosamine (Neu5Acα2-3Galβ1-4GlcNAc).

GDP-L-fucose can be prepared using recombinant enzymes of either of the two known biosynthetic routes. In principle, the recombinant enzymes can be produced in any prokaryotic or eukaryotic cell that has been transformed with a vector comprising a sequence coding for GMD, GFS, FK or PP. GDP-L-Fuc can then be produced by reacting the appropriate substrate with the enzyme converting it. The enzyme may be in the form of a cell lysate, or it may be isolated therefrom and further purified. The two enzymes needed to convert GDP-Man or L-Fuc, respectively, into GDP-L-Fuc may even be produced in different organisms and separately added to the reaction mixture. Preferably, the host cell is yeast or mold, especially *Saccharomyces cerevisiae.* Yeast and mold cells contain intrinsic GDP-D-Man, which can serve as substrate for the biosynthesis of GDP-L-Fuc via the *de novo* pathway.

A very convenient way for the recombinant production of GDP-L-Fuc is to construct a double vector, i.e. a vector comprising the two genes needed for the *de novo* pathway or the two genes needed for the salvage pathway. A host cell transformed with such a double vector can produce a chimeric enzyme having the activity of both enzymes. A yeast or mold cell comprising both *gmd* and *gfs* genes can produce GDP-L-Fuc without external addition of GDP-D-Man.

GDP-L-fucose serves as fucose donor for the fucosylation of glycans by fucosyltranseferases. According to the present invention, fucosylated glycans are prepared by reacting GDP-L-Fuc with recombinantly produced α1,3-fucosyltransferase, preferably obtained from rat or bacteria. The host cell can be any appropriate prokaryotic or eucaryotic cell, including animal cells.

The glycans to be fucosylated are preferably polylactoseamines, which are converted into sialyl Lewis x (sLex) sugars. The α-1,3-fucosyltransferase transfers fucose in α-1,3-position to a GlcNAc-sugar. GlcNAc can be part of the polylactoseamine backbone e.g. Galβ1,4GlcNAcβb1,3-Galβ1,4GlcNAc, which in turn may be part of a glycoprotein or a glycolipid. In other words, the process of the present invention can be used for fucosylating sugars as such, including naturally occurring and synthetically produced ones, or for fucosylating glycoproteins and glycolipids. All these fucoglycosylated compounds have potential use as selectin inhibitors. They may be further formulated into pharmaceutical compositions.

The present invention provides a number of novel genes which are useful in the claimed process. Said genes comprise a DNA sequence encoding a certain enzyme from a certain organism. The sequenced DNA sequences are given as SEQ ID No. 1 - 8. The present invention is especially directed to the enzyme coding parts (starting at ATG) of these sequences. However, it is to be understood that the invention is not limited to the exact sequences, but it includes any sequences that encode the stated enzymes. The invention also includes the complementary sequences, hybridizing sequences and sequences which, but for the degeneracy of the genetic code, would hybridize to them.

The DNA sequences are incorporated into vectors together with appropriate promotors and selection markers. Suitable promotors are e.g. GAL promotors. Antibiotic resistance or essential amino acids may be used as selection markers. The vectors are transformed or transfected into host cells and the recombinant host cells are then cultivated under conditions allowing expression of the encoded enzymes. The enzymes are then recovered from the cells, or the cell lysate is used directly, to catalyze a desired enzymatic reaction. The enzyme or the cell lysate comprising the enzyme is then contacted with its substrate to form the desired product. Alternatively, the enzyme reaction is carried out within the recombinant host cell, which thus directly produces the desired reaction product.

Assays for the determination of GDP-Fuc or fucosyltransferase facilitate the monitoring of the process of the invention. The assays are especially suitable for the determination of GDP-L-Fuc, but the same principle may also be applied to the determination of other sugar nucleotides. Correspondingly, the determination of fucosyltransferases is especially suited for the determination of α-1,3-fucosyltransferase, but the same principle may also be applied to the determination of α-1,2- or α-1,6-fucosyltransferases. Since fucosylation increases during infection and inflammation, the assays of the present invention can also be used in diagnosis. Increased amounts of GDP-Fuc and/or fucosyltransferases in the body indicate infection or inflammation diseases. The GDP-Fuc and fucosyltransferase activity may be determined in any tissue sample. Since fucosyltransferase is also secreted, its activity can further be determined in any body fluid sample, such as blood serum, plasma, spinal cord fluid, joint fluid, tears, saliva etc. The assays may also be used in determining the presence of the *gmd, gfs*, *fk* or *pp* genes or their mRNA or the corresponding enzymes.

According to one embodiment of the invention, the assay comprises incubating a sample suspected to contain GDP-fucose with a fucosyltransferase and a sLN-polyacrylamide-biotin conjugate to form a biotinylated fucosylated glycoconjugate (sLex conjugate); contacting the reaction mixture of the previous step with immobilized streptavidin to immobilize the biotinylated sLex conjugate; reacting the biotinylated sLex conjugate with a primary anti-sLex antibody, and then with a secondary europium-labelled antibody that recognizes the primary antibody; and detecting any time-resolved fluorescence as a measure of GDP-fucose in the sample.

Alternatively, the assay comprises incubating a sample suspected to contain fucosyltransferase with GDP-L-fucose and a sLN-polyacrylamide-biotin conjugate to form a biotinylated fucosylated glycoconjugate (sLex conjugate); contacting the reaction mixture of the previous step with immobilized streptavidin to immobilize the biotinylated sLex conjugate; reacting the biotinylated sLex conjugate with a primary anti-sLex antibody, and then with a secondary europium-labeled antibody that recognizes the primary antibody; and detecting any time-resolved fluorescence as a measure of fucosyltransferase in the sample.

A test kit useful in the assay above can contain e.g. the fucosyltransferase or GDP-Fuc, the sLN-polyacrylamide-biotin conjugate, optionally immobilized streptavidin, the primary anti-sLex antibody, and the labelled secondary antibody, and optionally reaction and washing solutions.

According to another embodiment of the invention, the assay comprises immobilizing a biotinylated Lex-polyacrylamide conjugate onto a carrier coated with streptavidin; adding a sample suspected to contain GDP-fucose, and europium-labeled fucose-specific lectin AAL, incubating and washing; and detecting any time-resolved fluorescence, whereby a decrease in the fluorescence indicates the amount of GDP-Fuc present in the sample.

A test kit useful in this assay may comprise biotinylated Lex-polyacrylamide conjugate, optionally immobilized streptavidin and labeled fucose-specific lectin AAL. It may also comprise appropriate reaction and washing solutions.

### GMD and GFS

Here we describe the molecular identification, cloning and expression strategies of identifying novel genes coding for this pathway and their co-expression in yeast cells such as *S*. *cerevisiae.* Novel *gmd* and *gfs* (also termed *gmers,* or *fx)* genes from *H*. *pylori* were identified, cloned, sequenced and functionally expressed together in a double-vector in *S*. *cerevisiae.* The corresponding *E.coli* genes can also be succesfully expressed in this yeast system. The great benefit of this yeast cell approach is the fact that the expression host has spontaneously very high levels of GDP-D-Mannose, the precursor of GDP-L-Fuc synthesis, in the cytosol and furthermore, it lacks primarily the genes and enzymes involved in the fucose metabolism.

Also two chimeric molecules, namely GMD-GFS and GFS-GMD, were generated by PCR and the enzymatic activity could be shown even with this approach. The great benefit of this approach is that this way the otherwise unstabile GMD is better preserved and furthermore, only a one-step purification of the enzyme(s) is needed before it can be coupled to a matrix, etc.

We have also probed whole-genome gene-chips of *S. cerevisiae* having either or both *gmd* and *gfs* genes transfected. By this approach we can identify the genome-wide expression patterns of genes and identify pathways that are disrupted due to the transfections. We are able to perform metabolic engineering to the yeast strains transfected with novel genes and further enhance the productivity of the GDP-L-Fuc.

Previous work has shown that *gmd* and *gfs* can be overexpressed in *H*. *pylori* and that when exogenous GDP-D-mannose is introduced into the reaction, GDP-L-fucose is synthesized. However, our work offers a major improvement to this approach as no exogenous expensive GDP-D-mannose needs to be added to the yeast cell lysates expressing GMD and GFS (GFS) enzymes.

The availability of GDP-D-mannose was shown to be a limiting factor for our yeast transformant expressing *gmd* as well as *gfs* genes. In yeast cells GDP-D-mannose is synthesized in the cytoplasm and further transported to the lumenal space of Golgi apparatus by a specific antiporter system that involves exchange with guanosine 5'-monophosphate (GMP). Thus, as the recombinant enzymes are expressed in the cytosolic compartment of the yeast, it might be beneficial to use mutant yeast cells, which have been characterized to have a defect in the GMP antiporter function leading to increased cytosolic GDP-D-mannose levels.

Taken together, we have shown here that bacterial *gmd* and *gfs* genes can be expressed as functional enzymes in *S*. *cerevisiae* and due to the inherent GDP-D-mannose synthesis in yeast cells they synthesize GDP-L-fucose. This approach was shown to be relatively effective as > 0.2 mg/l GDP-L-fucose was produced by specifically transfected yeast cells. It should also be noted that no optimization of the yeast cell culture systems has been performed so far to increase the yield. The GDP-L-fucose generated by this rapid route can be further converted to bioactive fucosylated glycans with relevant recombinant fucosyltransferases.

### FK and PP

The salvage pathway utilizes L-Fuc and converts it to GDP-L-Fuc via two enzymatic reactions catalyzed by fuco-1-kinase (FK) and GDP-L-fucosephosphorylase (PP). Here we describe the molecular identification, cloning and expression strategies of identifying novel genes coding for the first step of the salvage pathway, i.e. fuco-1-kinase. In this pathway fukokinase utilizes L-Fuc and conversts it to L-Fuc-1-P. We have first identified the FK enzyme by using the three known peptide sequences known from the enzyme and generating *in silico* probes from them. With these probes and with the help of both human and murine EST cDNA databanks, we were able to pull out the putative human and murine *fk* genes. The murine *fk* gene can then be expressed in *S.cerevisiae* and other cells, such as bacterial, yeast, insect and mammalian host by novel multiplatform vectors (such as gateway), and the enzymatic activity was demonstrated in a system which normally is devoid of background fucose metabolism. The tissue expression pattern of the novel *fk* gene was analysed by Northen blotting and relatively high levels of expression were detected in different organs.

The second enzyme in this pathway is the PP enzyme (GDP-L-fucose-pyrophosphorylase), which converts the Fuc-1-P into GDP-L-Fuc. The human PP (hPP) enzyme has previously been sequenced. Now we have sequenced the gene coding for murine and rat PP. Using sequence homology we were able to PCR the murine and rat *pp*'s from the kidney cDNA libraries. We show that there are at least two variants of this gene in the mammalian genome and, after cloning and sequencing the *pp* gene, we have also expressed it in the *S. cerevisiae*. Preferably these two genes, *fk* and *pp* are expressed in a double vector in order to generate a chimeric molecule and thereby increase the efficacy to synthesize GDP-L-Fuc.

### Transferases

### α-1,3-Fucosyltransferase enzymes

Fucosyltransferase transfers GDP-L-Fuc (donor) to a growing glycan chain. We have cloned, sequenced and expressed a bacterial α-1,3-fucosyltransferase enzyme from *H*. *felis*. Furhermore, we have cloned, sequenced and expressed a novel rat α-1,3-fucosyltransferase (FucT-VII) enzyme, which is crucial in the synthesis of sialylated glycans. Both of these genes code enzymes that can be used to α-1,3-fucosylate glycan and glycoproteins with the help of GDP-L-fucose.

### Assays

To this end, we have developed high-through put assays for the identification of GDP-L-Fuc in cell extracts. We have two assays, the first one detects smaller amounts of GDP-L-Fuc present in the sample and relays on the activy of a specific fucosyltransferase enzyme, and the newly synthetized glycans are detected with relevant monoclonal antibodies in a time-resolved immunofluorometric assay and the second relays on the specific detection of GDP-L-Fuc by fucose-binding activity of AAL lectin.

We have recently developed rapid and sensitive high-throughput assays for glycosyltransferases and glycosidases, which employ microplate assay technology and time-resolved fluorometric detection. In the detection of GDP-Fuc, we utilized α-1,3-fucosyltransferase assay, in which the limiting factor was the presence or absence of GDP-Fuc in the sample. The α-1,3-fucosyltransferase reaction converting sialyllactosamine (sLN) to sialyl Lewis x (sLex)-tetrasaccharide was performed in the solutionphase, after which the biotinylated glycoconjugate was immobilized onto streptavidin-coated microplate. The fucosylated reaction product sLex was then detected by time-resolved immunofluorometry. This assay combines the advantages of solution-phase enzymatic reaction and solid-phase detection technology, being versatile and capable of simultaneous processing of multiple samples.

A faster low-cost method based on inhibition of europium-labelled fucose-specific *Aleuria aurantia* lectin (AAL) was also developed for measuring GDP-Fuc concentration. The lectin-based assay is less sensitive than the enzymatic assay, but as a cheap and rapid method it is well-suited for optimizing production of GDP-Fuc in yeast. As a large variety of different lectins, glycosyltransferases, and glycoconjugates are commercially available, the newly developed methods are applicable to the analysis of many different nucleotide sugars and glycosyltransferases as well.

The use of conventional chromatographic and radiochemical methods in quantitating GDP-Fuc is complicated by the fact that different nucleotide sugars are similar in structure and the cellular concentration of GDP-Man is high in yeast. Furthermore, in optimizing the conditions for the production of GDP-Fuc, large amounts of samples have to be monitored. Thus, we took advantage of stereospecific recognition of fucose by either α-1,3-fucosyltransferase enzyme or a fucose-specific lectin, the functions of which are easy to detect in microplate assays. The advantages of the new assays include ease of procedure and capability of simultaneous processing of multiple samples.

In the enzyme assay, solution-phase enzymatic reaction and solid-phase detection were performed. In the FucT assay earlier published, we used immobilized acceptor, but later we have noticed that a soluble acceptor is clearly better for this enzyme. The reason for this is unknown, but probably it is kinetically advantageous that all components of the enzymatic reaction are in the solution-phase.

As an end point assay, FucT assay is more sensitive and precise in the determination of GDP-Fuc concentration than the lectin inhibition assay, in which the affinity of the lectin to fucose determines the sensitivity. Thus the benefits of time-resolved fluorometric detection are better seen in FucT assay. Time-resolved fluorometry is based on the unique fluorescence properties of some lanthanide chelates and has proved to provide remarkably high sensitivity and a wide range of measurements in noncompetitive assays. Compared to the enzymatic assay, the lectin inhibition assay works with higher concentrations of GDP-Fuc, and the concentrations of GDP-Fuc in the samples we used in this study were only slightly over detection limit. However, in optimizing the production of GDP-Fuc, better yields will probably be achieved and this method is a promising tool because the components of the assay are cheap and incubations take under 1 hour.

The two assays complement each other; the enzymatic assay is suitable for the detection of minute amounts of GDP-Fuc, whereas the robust and cheap lectin assay requires micromolar concentrations of GDP-Fuc before it can be used. Both assays work well with crude cell lysates, but if necessary, GDP-Fuc can be purified with *Aleuria aurantia* lectin affinity chromatography. The enzyme-based method can be used in measuring of both *de novo* and salvage reaction pathways, but the lectin-based method is applicable only with the *de novo* pathway, as free fucose would disturb the assay.

In conclusion, the methods we have developed should prove very useful in monitoring the production of GDP-Fuc and in the analysis of fucose metabolism in cells. These assays are attractive alternatives to the currently used methods especially in screening of large numbers of crude biological samples. The methods used in this study can also be easily adapted to the analysis of other enzymes in glycobiology.

The following examples illustrate the present invention.

### Example 1

### Functional expression of Escherichia coli enzymes synthesizing GDP-L-fucose from inherent GDP-D-mannose in Saccharomyces cerevisiae

### Preparation of gene constructs

The *gmd* and *wcaG* coding regions were amplified from *E.coli* K-12 wca gene cluster (accession U38473). Advantage polymerase (Clontech, Palo Alto, CA, USA) was used with the primer set
5'AAGAACTCGAGTCAAAAGTCGCTCTCAT3' (creating a Xhol-site) and
5'TTATAAGCTTTTATGACTCCAGCGCGA3' (creating a Hind III-site) to amplify gmd (nucleotides 8662-9780) as well as primer set
5'CAAGAAAGATCTCAAGTAAACAACGAGTTTTT3'(creating a Bgl II-site) and
5'TTATGAGCTCTTACCCCCGAAAGCGGTC3' (creating a Sac I-site) to amplify wcaG (nucleotides 9786-10748). Both PCR-products were cloned into PCR-blunt II TOPO (Zero-blunt-TOPO; PCR-kloning kit, Invitrogen, Groningen, The Netherlands). Both inserts were digested out of PCR-blunt II TOPO and transferred to pESC-leu vector (Stratagene, La Jolla, Ca,USA). The gmd-gene was subcloned under P_{GAL}1-promoter inframe with c-myc-epitope and the wcaG-gene under P_{GAL}10-promoter inframe with FLAG-epitope. The construct of this vector is shown in Figure 2. Also vectors containing only either of the inserts were prepared. The constructs were confirmed by sequencing (ABI 310, PE Biosystems, Fostercity CA, USA).

### Transformation into S. cerevisiae

The pESC-leu vectors not containing any genes, containing either *gmd* or *wcaG* or both of the genes were transformed into YPH 499 and YPH 501 yeast host strains by lithium acetate method following the instructions of the manufacturer (Stratagene). Transformants were selected using leucine dropout plates.

### Selection of yeast transformants

Several transformants growing on leucine dropout plates were picked up and grown in 25 ml of dextrose containing selective synthetic dropout (SD) media overnight. The GAL1 and GAL10 promoters were repressed when transformed yeast cells were grown in dextrose containing SD media and induced when the cells are changed to grow in galactose containing SG media. In a typical experiment the yeast cell mass was increased by growing them in SD media overnight and the expression of transformed genes was induced for another 24 h by changing the carbon source of the media from dextrose to galactose. After centrifugation the yeast cells were grown another 24 h in the same volume in synthetic galactose containing dropout (SG) media and the OD₆₀₀ was measured. 1x10⁹ cells were spinned down, suspended in 0.5 ml of breaking buffer containing 1% TX-100 and 10% glycerol and the cells were lysed mechanically by vortexing with glass beads (1/3 volume). After centrifugation the cell lysates were subjected to protein analysis as well as to enzymatic activity assays. 25 µg of total protein was used in Western blots and 0.3-0.4 mg/ml in the activity assys.

### Expression of E.coli gmd and wcaG in yeast

Expression of recombinant proteins was detected on the RNA and the protein level. Total RNA (15mg) was extracted from double transformant yeast cells as well as from negative controls and broken mechanically with glass beads and subjected to Northern blot analysis as described before [Mattila P, Joutsjärvi V, Kaitera E, Majuri M, Niittymäki J, Maaheimo H, Renkonen R, and Makarow M: Targeting of active rat a2,3-sialyltransferase to the yeast cell wall by the aid of the hsp150Dcarrier. Towards the synthesis of sLex decorated L-selectin ligands. Glycobiology 1996, 6:851-859]. The blots were probed with PCR products amplified from E.coli K-12 wca gene cluster. The expression of GMD and GFS (GFS) was studied in Western blot using the antibodies against c-myc and FLAG-epitopes, respectively. Chemiluminescence (ECL, Amersham) was used as a detection method according to manufacturer's instructions.

### Determination of GDP-L-fucose synthesis

The presence of GDP-L-fucose was assayed by using yeast cell lysate as a source of GDP-L-fucose in a fucosyltransferase reaction converting sialyl-N-acetyllactosamine (sLN) to sLex. The reaction mixture included fucosyltransferase VI (FucTVI 25 µU, Calbiochem; San Diego, CA), yeast cell lysate diluted 1:20 as a fucose donor, sLN-polyacrylamide-biotin conjugate (Syntesome; Moscow, Russia) as a fucose acceptor, 50 mM MOPS-NaOH (pH 7.5), 6 mM MnCl₂, 0.5% Triton X-100, 0.1% BSA, and 1 mM ATP. After 1 h incubation in +37°C 50 µl aliquots of the reaction mixtures were transferred to microtitration strips coated with streptavidin (Wallac; Turku, Finland) to immobilize the biotinylated glycoconjugate. The fucosylated reaction product sLex was then detected and quantified by time-resolved fluorometry (Wallac) using anti-sLex primary antibody KM-93 (Calbiochem) and europium-labelled (DELFIA Eu-labelling kit: Wallac) anti-mouse IgM secondary antibody (Sanbio; Uden, The Netherlands) as described before [Räbinä J, Smithers N, Britten CJ, and Renkonen R: A Time-Resolved Immunofluorometric Method For the Measurement Of Sialyl Lewis X-Synthesizing Alpha-1,3-Fucosyltransferase Activity. Analytical Biochemistry 1997, 246:71-78].

### Purification of GDP-L-fucose

For a large-scale GDP-L-fucose synthesis, one transformant containing both *gmd* and *wcaG* genes was grown 24 h in 750 ml of selective SD media and another 24 h in selective SG media. The cells were collected when OD₆₀₀ was 3.7 and the pellet was suspended in concentration of 2x10⁹ cells/ml (total volume 45 ml) of breaking buffer containing 1% TX-100 and 10% glycerol and lysed with glass beads. After vigorous 30 min vortexing the glass beads and cell debris were centrifuged and the clear lysate was filtrated through YM-10 centricon column (Millipore Corporation, Bedford, MA, USA) o/n +4°C according to the manufacturer's instructions.

GDP-L-fucose synthesized in yeast cells was then purified in two chromatographic steps. Lectin affinity chromatography was performed on a small column (diameter 0.5 cm) of agarose-bound *Aleuria aurantia* lectin (2 ml; Vector laboratories, Burlingame, CA). The column was equilibrated with 10 mM HEPES buffer, pH 7.5, containing 0.15 M NaCI and 0.02% NaN₃. After application of yeast cell lysate (4 ml) into the column, the elution was performed with 4 ml of the equilibration buffer followed by 4 ml of the buffer containing 25 mM L-fucose. Fractions of 1 ml were collected and assayed for the presence of GDP-L-fucose.

For desalting GDP-L-fucose and removal of the haptenic sugar, size-exclusion HPLC on a Superdex Peptide HR 10/30 column (Pharmacia, Sweden) was used. The elution was performed at 1 ml/min using 50 mM NH₄HCO₃ and the effluent was monitored with a UV detector at 254 nm. The amount of GDP-L-fucose was calculated from peak areas by reference to external standard (GDP-L-fucose, Calbiochem).

### Matrix-assisted laser desorption/ionization time-of-flight mass spectrometry

Maldi-TOF mass spectrometry was performed with a Biflex mass spectrometer (Bruker Daltonics, Germany). Analysis was performed in the negative-ion linear delayed-extraction mode, using 2,4,6 - trihydroxyacetophenone (THAP, Fluka Chemica) as the matrix as described. External calibration was performed with THAP matrix dimer and sialyl Lewis x beta-methylglycoside (Toronto Research Chemicals, Canada).

### Results

### Preparation of gmd and wcaG gene constructs and screening of S. cerevisiae transformants

In order to induce GDP-L-fucose synthesis in *S. cerevisiae* the *E*. *coli gmd* coding for GDP-D-mannose-4,6 dehydratase (GMD) activity and *wcaG* coding for GDP-4-keto-6-deoxy-D-mannose epimerase/reductase, i.e GFS (GFS) activity, were inserted into pESC-leu-vector under GAL1 and GAL10 promoters, respectively (Fig. 2). The *gmd* was in frame with the c-myc -epitope and the *wcaG* with the FLAG -epitope as revealed by DNA sequencing.

The vector controls without any genes, single constructs containing only either *gmd* or *wcaG* or double constructs containing both genes were transformed into two yeast host strains YPH 499 and YPH 501. Several transformants of both host strains capable of surviving and proliferating on selective leucine deficient drop-out plates were screened for expression of *gmd* and *wcaG* -genes on the RNA level as well as the corresponding enzymes, GMD and GFS, on the protein level.

### Expression of gmd and wcaG in S. cerevisiae

In Northern blot analysis a 1.3 kb transcript was detected for *gmd* in both yeast strains YPH 499 and YPH 501 transformed with either only E. coli *gmd* or both *gmd* and *wcaG.* When the presence of the corresponding enzyme, GMD was assayed with the c-myc antibody in Western blots, a 48 kD protein band was detected in the single transfectants containing only the *gmd* gene or double transfectants containing both the *gmd* and *wcaG* genes. Concomitantly the mock transformants showed no signals in either Northern or Western blots.

Correspondingly, a 1.1 kb band for *wcaG* was present in the Northern blot analysis from both yeast strains YPH 499 and YPH 501 transformed with either only E. coli *wcaG* or both *gmd* and *wcaG* genes. The corresponding enzyme coded by *wcaG* was expressed as a 40 kD band as detected with anti-FLAG -antibody. Again, no relevant bands were detected in mock controls or with control antibodies.

### Enzymatic activity of E.coli GMD and GFS in yeast

The intermediate products of the *de novo* pathway from GDP-D-mannose to GDP-L-fucose (GDP-4-keto-6-deoxy-D-mannose and GDP-4-keto-6-deoxy-L-galactose) are known to be labile and not feasible to monitor in a high throughput screening approach. Thus we used a microwell plate assay developed in our laboratory for measuring the α-1,3-fucosylation. In this assay biotinylated polyacrylamide conjugated sLN and recombinant α-1,3-fucosyltransferase VI were added to yeast cell lysates either not containing (mock- or only single transformants with either *gmd* or *wcaG* ) or containing GDP-L-fucose, i.e. double transformants with both the *gmd* and *wcaG* genes. The readout of this assay was the turnover of sialyllactosamine to sLex detected by specific antibodies and time-resolved immunofluorometry. As the relevant acceptor and enzyme were always present in this assay, the only limiting factor in the generation of sLex from sLN was the presence or absence of GDP-L-fucose.

Both the mock-transfectants as well as the yeast cells transformed separately only with *gmd* or *wcaG* were unable to synthesize GDP-L-fucose resulting in background values in the enzymatic assay. When the lysates of *gmd* and *wcaG* expressing yeast clones were mixed together, the synthesis of sLex was increased from 0% (background level) to 10.7% (YPH 499) or to 16.6% (YPH 501) in a one-hour assay. To prove that these genes could be transformed into the same yeast cell host, *gmd* was expressed under GAL1 promoter and *wcaG* under GAL10 promoter within the same pESC-leu vector in either YPH 499 or YPH 501. These double transfectants were able to produce enough GDP-L-fucose to enable 3.4% (YPH 499) and 5.1% (YPH 501) of the sLN acceptor to be converted to sLex in a one-hour assay.

To analyse if the amount of the inherent, yeast produced GDP-mannose was a limiting factor for the GDP-L-fucose synthesis in the double transformed yeast strains, external GDP-D-mannose was added (0-500 µM) to the yeast cell lysates before incubation with relevant sLN acceptor and recombinant α-1,3-fucosyltransferase. Addition of external GDP-D-mannose increased the amount of GDP-L-fucose synthesis 3-fold, i.e. from 3.4% to 12.5% (YPH 499 ) or from 5.1% to 12.5% (YPH 501). We also showed that the addition of exogenous NAPDH did not further enhance the synthesis of GDP-L-fucose suggesting that the concentration of this cofactor in the yeast cell lysates does not represent a limiting factor in this assay.

### Purification and MALDI-TOF MS of GDP-L-fucose

To further confirm the synthesis and to quantitate the amount of GDP-L-fucose produced in the *gmd* and *wcaG* expressing yeast cells, *Aleuria aurantia* lectin affinity chromatography was used to purify the product. As monitored with the enzymatic α-1,3-fucosyltransferase assay, most of the GDP-L-fucose bound to the lectin column and was eluted with addition of exogenous L-fucose. The peak fraction was further purified by size-exclusion HPLC, in which the retention times of both the purified product and commercial GDP-L-fucose was 16.2 min. As compared to external standard (GDP-L-fucose), the amount of GDP-L-fucose after purification was 3 mg per 1 ml of the original yeast cell lysate, corresponding to 0.2 mg/l of GDP-L-fucose in the original yeast cell culture.

This HPLC-purified product was then subjected to a MALDI-TOF MS analysis. The product purified from the double transfected yeast cells comigrating with the commercial GDP-L-fucose gave a single peak at *m/z* 588.04, (calculated *m/z* for [M-H]⁻ of GDP-L-fucose is 588.08). A single peak seen in the appropriate area in MALDI-TOF MS not only indicates that the product is GDP-L-fucose, but also shows that it was free of other nucleotide sugars.

### Example 2

### Identification, cloning and functional expression of novel gmd and gfs genes from Helicobacter pylori

Using *in silico* cloning we were able to identify the putative gmd and gfs genes from the whole genome of the published *H. pylori* J99 (Genbank accession number AE001443). By sequence alignment the putative *gmd* gene was identified as jhp0038 in J99 and hp0044 (SEQ ID No. 1) in 26695, also known as *rfb*D. Similarly the *gfs gene* was suggested to be the jhp0039 in J99, hp0045 (SEQ ID No. 2), i.e. *nol*K in 26695, which has been named as *wbc*J (McGowan)

In order to induce GDP-L-fucose synthesis in *S*. *cerevisiae,* the *H. pylori gmd* coding for GDP-D-mannose-4,6 dehydratase (GMD) activity and *gfs* coding for GDP-4-keto-6-deoxy-D-mannose epimerase/reductase, i.e GFS activity, were inserted into pESC-leu-vector under GAL1 and GAL10 promoters, respectively. The *gmd* was in frame with the c-myc-epitope and the *gfs* with the FLAG-epitope as revealed by DNA sequencing. The vector construct was equivalent to that shown in Fig. 2.

### Preparation of gene constructs

The *gmd* and *gfs* coding regions were amplified from an *H*. *pylori* gene cluster (accession U38473). Advantage polymerase (Clontech, Palo Alto, CA, USA) was used with the primer set
5'AAGAACTCGAGTCAAAAGTCGCTCTCAT3' (creating a Xhol-site) and
5'TTATAAGCTTTTATGACTCCAGCGCGA3' (creating a Hind III-site) to amplify *gmd* (nucleotides 8662-9780) as well as primer set
5'CAAGAAAGATCTCAAGTAAACAACGAGTTTTT3'(creating a Bgl II-site) and
5'TTATGAGCTCTTACCCCCGAAAGCGGTC3' (creating a Sac I-site) to amplify gfs (nucleotides 9786-10748). Both PCR-products were cloned into PCR-blunt II TOPO (Zero-blunt-TOPO; PCR-kloning kit, Invitrogen, Groningen, The Netherlands). Both inserts were digested out of PCR-blunt II TOPO and transferred to pESC-leu vector (Stratagene, La Jolla, Ca,USA). The gmd-gene was subcloned under P_{GAL}1-promoter inframe with c-myc-epitope and the gfs -gene under P_{GAL}10-promoter inframe with FLAG-epitope. Also vectors containing only either of the inserts were prepared. The constructs were confirmed by sequencing (ABI 310, PE Biosystems, Fostercity CA, USA).

The vector controls without any genes, single constructs containing only either *gmd* or *gfs* or double constructs containing both genes were transformed into two yeast host strains YPH 499 and YPH 501. Several transformants of both host strains capable of surviving and proliferating on selective leucine deficient drop-out plates were screened for expression of *gmd* and *gfs* -genes on the RNA level as well as the corresponding enzymes, GMD and GFS, on the protein level.

### Expression of gmd and gfs in S. cerevisiae

In Northern blot analysis a 1.3 kb transcript was detected for *gmd* in both yeast strains YPH 499 and YPH 501 transformed with either only *H.pylori gmd* or both *gmd* and *gfs.* When the presence of the corresponding enzyme, GMD, was assayed with the c-myc antibody in Western blots, a 48 kD protein band was detected in the single transfectants containing only the *gmd* gene or double transfectants containing both the *gmd* and *gfs* genes. Concomitantly the mock transformants showed no signals in either Northern or Western blots.

Correspondingly, a 1.1 kb band for *gfs* was present in the Northern blot analysis from both yeast strains YPH 499 and YPH 501 transformed with either only E. coli *gfs* or both *gmd* and *gfs* genes. The corresponding enzyme coded by *gfs* was expressed as a 40 kD band as detected with anti-FLAG-antibody. Again, no relevant bands were detected in mock controls or with control antibodies.

### Enzymatic activity of H. pylori GMD and GFS in yeast

The intermediate products of the *de novo* pathway from GDP-D-mannose to GDP-L-fucose (GDP-4-keto-6-deoxy-D-mannose and GDP-4-keto-6-deoxy-L-galactose) are known to be labile and not feasible to monitor in a high throughput screening approach. Thus we used a microwell plate assay developed in our laboratory for measuring the α-1,3-fucosylation. In this assay biotinylated polyacrylamide conjugated sLN and recombinant α-1,3-fucosyltransferase VI were added to yeast cell lysates either not containing (mock or only single transformants with either *gmd* or *gfs*) or containing GDP-L-fucose, i.e. double transformants with both the *gmd* and *gfs* genes. The readout of this assay was the turnover of sialyllactosamine to sLex detected by spesific antibodies and time-resolved immunofluorometry. As the relevant acceptor and enzyme were always present in this assay, the only limiting factor in the generation of sLex from sLN was the presence or absence of GDP-L-fucose.

Both the mock-transfectants as well as the yeast cells transformed separately only with *gmd* or *gfs* were unable to synthesize GDP-L-fucose resulting in background values in the enzymatic assay. When the lysates of *gmd* and *gfs* expressing yeast clones were mixed together, the synthesis of sLex was increased from 0% (background level) to over 15 % in a one-hour assay. To prove that these genes could be transformed into the same yeast cell, host *gmd* was expressed under GAL1 promoter and *gfs* under GAL10 promoter within the same pESC-leu vector in either YPH 499 or YPH 501. These double transfectants were able to produce enough GDP-L-fucose to enable the sLN acceptor to be converted to sLex.

### Example 3

### Cloning and functional expression of murine and human L-fucokinase (FK)

### In silico and in vitro cloning of L-fucokinase

L-fucokinase (FK) has previously been purified to apparent homogeneity from pig kidney cytosol by Park et. al. 1998. The purified porcine enzyme has been subjected to endo-Lys-C digestion, followed by peptide isolation and their amino acid sequencing. This approach has yielded three peptides, with the following sequences:
peptide 1; VDFSGGWSDTPPLAYE
peptide 2; (T)(G)IRDWDLWDPDTP(P)(T)ER
peptide 3; LSWEQLQPCLDR

*In silico* parts of the present work included BLAST search in a HTGS database and electronic Northern blots in the mouse EST database. *In vitro* PCR reactions to clone the murine *fk* gene were performed with probes identified in silico to match both putative human and murine sequences. The constructs were confirmed by sequencing (ABI 310, PE Biosystems, Fostercity CA, USA).

### Expression of murine FK protein

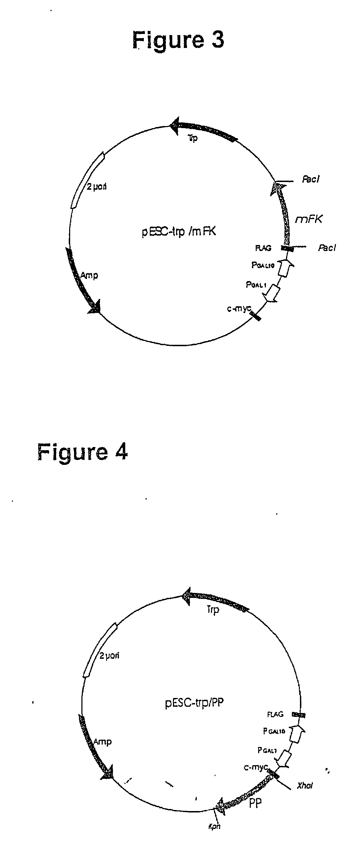

For protein expression the coding sequence of mouse FK (SEQ ID No. 3) nt 4-2388 was amplified with Advantage polymerase (Clontech, Palo Alto, CA, USA) with the primer set SFKsc5':
5'CCTTAATTAAGAGCAGTCAGAGGGAAGTCA3' (creating the Pad site) and SFKsc3':5'CCAGGGCCTTGCTGATTAATTAAGG3' (creating the Pad site). PESC-trp vector was modified by digesting with BgIII and Sacl to remove the internal stop codon. The overhangs were filled with klenow polymerase and the vector was blunt end ligated into PCR-blunt II TOPO (Zero-blunt-TOPO; PCR-kloning kit, Invitrogen, Groningen, The Netherlands). The amplified *fk* sequence was inserted in Pad site and the orientation was verified by sequencing. The *fk*-gene was subcloned under P_{GAL}1-promoter under P_{GAL}10-promoter inframe with FLAG-epitope. The vector construction is shown in Fig. 3. The constructs were confirmed by sequencing (ABI 310, PE Biosystems, Fostercity CA, USA).

### Transformation into S. cerevisiae and selection of transformants

The pESC-leu vectors either containing or not containing the *fk* gene were transformed into YPH 499 and YPH 501 yeast host strains by lithium acetate method following the instructions of the manufacturer (Stratagene). Transformants were selected using leucine dropout plates.

Several transformants growing on leucine dropout plates were picked up and grown in 25 ml of dextrose containing selective synthetic dropout (SD) media overnight. The GAL10 promoters were repressed when transformed yeast cells were grown in dextrose containing SD media and induced when the cells were changed to grow in galactose containing SG media. In a typical experiment the yeast cell mass was increased by growing them in SD media overnight and the expression of transformed genes was induced for another 24 h by changing the carbon source of the media from dextrose to galactose. After centrifugation the yeast cells were grown another 24h in the same volume in synthetic galactose containing dropout (SG) media and the OD₆₀₀ was measured. 1x10⁹cells were spinned down, suspended in 0.5 ml of breaking buffer containing 1% TX-100 and 10% glycerol and the cells were lysed mechanically by vortexing with glass beads (1/3 volume). After centrifugation the cell lysates were subjected to protein analysis as well as to enzymatic activity assays.

### Results

### In silico and in vitro cloning of human and murine fk genes

On the basis of the previously published three peptide sequences we performed a Blast search in a HTGS database containing unfinished human high throughput genomic sequences. The putative human *fk* gene was found to be located in chromosome 16 (accession AC012184, Genbank). The genomic sequence (nucleotides 34031-64743) was analyzed in a gene structure prediction server (GENSCAN). The length of the corresponding putative human *fk* cDNA was 2469 bp (SEQ ID No. 4), and the predicted corresponding FK protein of 823 amino acids.

When the predicted sequence of the human FK protein was used as a "probe" against mouse EST database, two EST sequences were found. The first EST sequence (accession number Al286399) had 91% identity on the nucleotide level with the 5' and the other sequence (accession number AA509851) had 88% identity on the nucleotide level with the 3'end of the putative human sequence. This electronically identified putative murine *fk* sequence was then verified by PCR amplifying cDNA using the following primers: a 5'primer mFK 5'(nucleotides 60-81 in AI286399) and 3'primer mfk stop (nucleotides 124-141 in AA509851).

The PCR reaction revealed a product of expected size and this was further sequenced.

### Expression of the murine FK protein in yeast cells

The yeast cells (*S.cerevisiase* and *Pichia pastoris*) were selected as expression hosts because these cells are devoid of their own fucose metabolism and thus allow perfect experimental conditions. For protein expression the coding sequence of mouse *fk* (nt 4-2388) was amplified with the primer set SFKsc5': 5'CCTTAATTAAGAGCAGTCAGAGGGAAGTCA3' (creating the Pad site) and SFKsc3':5'CCAGGGCCTTGCTGATTAATTAAGG3' (creating the Pacl site). PESC-trp vector was modified by digesting with Bglll and Sacl to remove the internal stop codon. The overhangs were filled with klenow polymerase and the vector was blunt end ligated. The amplified *fk* sequence was inserted in Pad site and the orientation was verified by sequencing.

The modified pESC-trp vector with the FLAG-epitope containing or not containing the mouse *fk* gene (Fig. 3) was transformed into *S*. *cerevisiae* strains YPH499 and YPH501. Positive clones were selected by Western blot (predicted weight of mFK is 87.5kD) using anti-FLAG-antibody.

In summary this example describes the cloning and stable expression of a murine *fk* gene catalysing the reaction converting L-fucose to L-fucose-1-P. Using the information of three short peptide sequences, which were previously characterized from the purified porcine FK protein, we were able to electronically clone the human *fk* gene (2469 bp) and it was found to be located in chromosome 16 (accession AC012184). The predicted human FK protein consisted of 823 amino acids. When the predicted sequence of the human FK protein was used as a "probe" against mouse EST database, two EST sequences were found. The first EST sequence (accession number Al286399) had 91% identity on the nucleotide level with the 5' and the other sequence (accession number AA509851) had 88% identity on the nucleotide level with the 3'end of the putative human sequence. This electronically identified putative murine *fk* sequence was then verified by PCR amplifying cDNA using the following primers: a 5'primer mFK 5'(nucleotides 60-81 in Al286399) and 3'primer mfk stop (nucleotides 124-141 in AA509851). This *fk* gene was present in several organs. When expressed in a *S*. *cerevisiae* or *P*. *pastoris* cell, devoid of background fucosylation we could identify an active enzyme. This enzymatic function was inhibited totally by excess of free fucose, 10% by excess of free arabinose while other monosaccharides had no effect.

### Example 4

### Cloning and expression of mouse and rat GDP-L-fucose pyrophosphorylase (PP)

### Cloning strategy for the GDP-L-fucose pyrophosphorylase

For the *in silico* and *in vitro* cloning of the murine and rat GDP-L-fucose pyrophosphorylase genes, the human 3144 bp cDNA coding GDP-L-fucose pyrophosphorylase (h*pp*, Pastuszak et. al accession number 017445 in GenBank) sequence was used as a probe in the murine and rat EST libraries. A perfectly matching probe for screening cDNA rat and murine kidney cDNA libraries was made after alignments and used as follows: MF2 (5'GAGTATTCTAGATTGGGGCCTGA3'nucleotides 37-59 in AA422658) as a forward primer and MR (5'GGAAAATGCGTGCAGTCCACA3' nucleotides 340-361 in AA422658) as a reverse primer. The PCR products were subcloned and subjected to sequencing. One clone was obtained from the murine library and three incomplete clones from the rat library. The missing 5'ends were obtained by RACE-PCR using gene specific primers
RACE 1(5'CTAGGCACTGAAGGGAACAAAGTGTCGATCCTC3')
RACE 2 (5'AGGCGTTGACTGTAGCCACCGGAGTGA3')
RACE 3(5'GACTCCAGGCTTCATGTGTAGGGGAAATCCACGTAC3') and
RACE 4(5'CACTGACAGTTCAATGTCATCTGCACAGGTGACCS').
cDNA alignments of mouse, rat and human *pp* sequences were performed. The mouse and rat sequences had SEQ ID No. 5 and SEQ ID No. 6, respectively.

Cloning the shorter transcript by 3'end RACE-PCR 3'end RACE PCR with two gene spesific primers MRrace1 (5'CTCTGTCCTAGCAAACACTGCTGTGCCG3') and MRrace2 (5'GAGGAACCTGAGCCTGTGGACTGCA3') and mouse and rat kidney cDNA libraries as template as well as Southern blot were performed to prove the excistence of two transcripts from a single gene.

### Expression of the mouse PP

For protein expression the coding sequence of m*pp* (nt 70-1842 of SEQ ID No. 5) was amplified with the primer set rmPPLH5U 5'CCGCTCGAGGAGACTCTCCGCGA3' (creating the Xhol site) and rmPPLHlyh 5'GGGGTACCTTAAGCTAGCATGTCTTGTACATC3' (creating the Kpnl site) and ligated to the *S*. *cerevisiae* expression vector pESC-trp (Figure 4).

pESC-trp vector was modified by digesting with BgIII and Sacl to remove the internal stop codon. The amplified *pp* sequence was inserted in Pacl site and the orientation was verified by sequencing. The *pp*-gene was subcloned under P_{GAL}1-promoter inframe with *c-myc-*epitope. The constructs were confirmed by sequencing (ABI 310, PE Biosystems, Fostercity CA, USA). The pESC-leu vector constructs either containing or not containign m*pp* gene were transformed into YPH 499 and YPH 501 yeast host strains by lithium acetate method following the instructions of the manufacturer (Stratagene). Transformants were selected using leucine dropout plates. Positive clones were selected by PCR showing the properly transfected cDNA clones and Western blot using anti-c-myc -antibody showing a protein of predicted weight of mouse/rat PP is 66kDa.

Several transformants growing on leucine dropout plates were picked up and grown in 25 ml of dextrose containing selective synthetic dropout (SD) media overnight. The GAL10 promoters were repressed when transformed yeast cells were grown in dextrose containing SD media and induced when the cells were changed to grow in galactose containing SG media. In a typical experiment the yeast cell mass was increased by growing them in SD media overnight and the expression of transformed genes was induced for another 24 h by chancing the carbon source of the media from dextrose to galactose. After centrifugation the yeast cells were grown another 24 h in the same volume in synthetic galactose containing dropout (SG) media and the OD₆₀₀ was measured. 1x10⁹cells were spinned down, suspended in 0.5 ml of breaking buffer containing 1% TX-100 and 10% glycerol and the cells were lysed mechanically by vortexing with glass beads (1/3 volume). After centrifugation the cell lysates were subjected to protein analysis as well as to enzymatic activity assays.

### Northern analysis and RT-PCR

The expression of *pp* transcripts in different rat and mouse tissues were analysed by Northern blot and RT-PCR. The Northern blot analysis revealed not only the expected 3.5kb band reported also with the hPP, but also an additional band of 1.7 kb. Because the expression level of *pp* was very low in certain tissues, the Northern results were verified by RT-PCR. On the basis of these experiments we concluded that rPP was expressed in every tissue of the panel, although at low levels.

### Enzymatic activity of PP

Biotinylated Lex- and sLN-polyacrylamide glycoconjugates were from Syntesome (Moscow, Russia). Anti-sLex antibody KM-93 (mouse IgM), a1,3-fucosyltransferase VI (FucTVI) and GDP-Fuc were from Calbiochem (San Diego, CA. Anti-mouse IgM (rat) antibody (Sanbio, Uden, The Netherlands) and streptavidin microtitration strips, enhancement solution, assay buffer, and wash concentrate were purchased from Wallac (Turku, Finland).

### Preparation of yeast cell extracts

Recombinant yeast strains transformed or not transformed with the *mpp* gene were first grown overnight in glucose-containing SD-media and then the expression of the foreing genes was stimulated by growing the recombinant strains in galactose containing SG-media for 24 h. 1x10⁹ cells were spinned down, suspended in 0.5 ml of lysing buffer containing 50 mM MOPS-NaOH (pH 7.0), 1% Triton X-100, and 10% glycerol and the cells were lysed mechanically by vortexing with glass beads.

### Measurement of GDP-Fuc with α-1,3-fucosyltransferase assay

The reaction mixture included the above mentioned yeast cells lysate containing the putative mPP enzyme, L-Fuc-1-P, sLN-polyacrylamide-biotin conjugate (1 µg/ml) as a fucose acceptor, fucosyltransferase (FucTVI 25 µU), 50 mM MOPS-NaOH (pH 7.5), 6 mM MnCl₂, 0.25% Triton X-100, 0.1% BSA, and 1 mM ATP. The reaction mixtures were incubated 1 h at +37°C on ultra low binding 96-plates (Costar, Cambridge, MA). After enzymatic reaction, aliquots of 50 µl of the reaction mixtures were transferred to microtitration strips coated with streptavidin to immobilize the biotinylated glycoconjugates. After immobilization (30 min), the fucosylated reaction product sLex was detected and quantified by time-resolved fluorometry (Wallac) and monoclonal antibodies. The anti-sLex primary antibody KM-93 (0.5 µg/ml) and europium-labelled anti-mouse lgM secondary antibody (0.6 µg/ml) were both diluted with DELFIA assay buffer and incubated 1 h at room temperature with vigorous shaking. Between and after incubations, the strips were washed six times with DELFIA wash solution. Finally, DELFIA enhancement solution (150 µl/well) was added and incubated 5 min at room temperature with slow shaking. The fluorescence was then measured with a time-resolved fluorometer (Wallac). For quantitation of GDP-Fuc in samples, defined concentrations of commercial GDP-Fuc diluted with sample buffer were used as standards.

### Results

### In silico and in vitro cloning of murin and rat pp genes

On the basis of the previously published data on the 3144 bp long human GDP-L-fucose pyrophosphorylase (h*pp*) cDNA sequence (Pastuszak I, Ketchum C, Hermanson G, Sjoberg EJ, Drake R, and Elbein AD: GDP-L-fucose pyrophosphorylase. Purification, cDNA cloning, and properties of the enzyme. J. Biol. Chem. 1998, 273:30165-74, accession number 017445 in GenBank) a 361 bp unknown mouse EST sequence (AA422658) was found, having 80 % homology with the human sequence between nucleotides 1257-1621. A perfectly matching pair of primers for screening rat and murine cDNA kidney cDNA libraries was designed by alignment of the murine and human genes.

### Detecting the enzymatic activity of mPP

We used our novel high-through put assay based on time-resolved immunofluorometry to detect the enzymatic activity of mPP in properly transfected yeast cell lysates. The expression of the *mpp* gene was stimulated by growing the recombinant strain in galactose containing SG-media for 24 h and lysed mechanically by vortexing with glass beads. The reaction mixture including the above mentioned yeast cells lysate containing the putative mPP enzyme was then incubated in the presence of L-Fuc-1-P and then sLN-polyacrylamide-biotin conjugate as a fucose acceptor and recombinant fucosyltransferase FucTVI were added for 1 hour. After enzymatic reaction, streptavidin immobilized the biotinylated glycoconjugates and the fucosylated reaction product sLex was then detected and quantified by time-resolved fluorometry with anti sLex monoclonal antibody. For quantitation of GDP-Fuc in samples, defined concentrations of commercial GDP-Fuc diluted with sample buffer were used as standards.

Our results show that the transformed yeast strains contain PP activity.

### Example 5

### Cloning of novel H. felis and rat α-1,3-fucosyltransferase genes

Fucosyltransferase transfers GDP-L-Fuc (donor) to a growing glycan chain. We have pulled out with low stringency cloning a bacterial α-1,3-fucosyltransferase enzyme from *H*. *fells,* cloned, sequenced and expressed it in *E.coli*, *S.cerevisiae* and Nawalma cells. The overall sequence homology and the conserved motif identifies it as a α-1,3-fucosyltransferase. The *H. felis* 1,3-fucosyltransferase gene has SEQ ID No. 8. Furhermore, we have also pulled out a novel rat α-1,3-fucosyltransferase (FucT-VII) enzyme, which is crucial in the synthesis of sialylated glycans, and cloned, sequenced and expressed in Nawalma cells.

The rat α-1,3-fucosyltransferase VII gene was cloned using cDNA from rat endothelial cells and primers designed based on homologies between human and murine genes. The cloned gene is 1910 bp long (SEQ ID No. 7) and the ORF is between nucleotides 194-1321 coding a 376 long amino acid. This gene was transfected to an expression vector pcDNA3 (with a neomycin resistance gene) and transfected to Namalwa cells by electroporation in order to establish a stable transfectant cell line with neomycin selection.

COS cells were transfected with the expression vector pCDNA3.1 (Invitrogen Inc. USA) comprising the rat FucT-VII gene or not comprising said gene (control). Fucosyltransferase activity was measured using sialyl lactosamine SA-alpha-2, 3Gal-beta-1, 4GlcNAc as acceptor whereby alpha-1,3-fucosylated sialyl Lewis x was formed. The specific fucosyltransferase activity of the COS/pCDNA3.1 vector was 0 pmol/mg/h and that of COS/pCDNA3.1 + rat FucT-VII was 87 pmol/mg/h. The results confirm that the cloned rat FucT-VII codes for alpha-1,3-fucosyltransferase.

### Example 6

**Two time-resolved fluorometric high-throughput assays for quantitation of GDP-L-fucose from cell lysates, tissue extracts, and body fluids**

### Materials and methods

Biotinylated Lex- and sLN-polyacrylamide glycoconjugates were from Syntesome (Moscow, Russia). Anti-sLex antibody KM-93 (mouse IgM), α1,3-fucosyltransferase VI (FucTVI) and GDP-Fuc were from Calbiochem (San Diego, CA) and fucose-specific lectin AAL was from Vector Laboratories (Burlingame, CA). Anti-mouse lgM (rat) antibody (Sanbio, Uden, The Netherlands) and AAL were labelled with europium according to the manual of the DELFIA Eu-labelling kit (Wallac, Turku, Finland). Streptavidin microtitration strips, enhancement solution, assay buffer, and wash concentrate were purchased from Wallac.

### Preparation of Yeast Cell Extracts

Recombinant yeast strains transformed with either GMD, GFS, or both of these genes were first grown overnight in glucose-containing SD-media and then the expression of the foreign genes was stimulated by growing the recombinant strains in galactose containing SG-media for 24 h. 1x10⁹ cells were spinned down, suspended in 0.5 ml of lysing buffer containing 50 mM MOPS-NaOH (pH 7.0), 1% Triton X-100, and 10% glycerol and the cells were lysed mechanically by vortexing with glass beads. The lysates and the mixture of single transformant lysates were incubated 2 h at +30°C.

### α-1,3-Fucosyltransferase Assay

The reaction mixture included fucosyltransferase (FucTVI 25 µU), a fucose donor (commercial GDP-Fuc or yeast cell lysate), sLN-polyacrylamide-biotin conjugate (1 µg/ml) as a fucose acceptor, 50 mM MOPS-NaOH (pH 7.5), 6 mM MnCl₂, 0.25% Triton X-100, 0.1% BSA, and 1 mM ATP. The reaction mixtures were incubated 1 h at +37°C on ultra low binding 96-plates (Costar, Cambridge, MA). After enzymatic reaction, aliquots of 50 µl of the reaction mixtures were transferred to microtitration strips coated with streptavidin to immobilize the biotinylated glycoconjugates. After immobilization (30 min), the fucosylated reaction product sLex was then detected and quantified by time-resolved fluorometry (Wallac) and monoclonal antibodies. The anti-sLex primary antibody KM-93 (0.5 µg/ml) and europium-labelled anti-mouse IgM secondary antibody (0.6 µg/ml) were both diluted with DELFIA assay buffer and incubated 1 h at room temperature with vigorous shaking. Between and after incubations, the strips were washed six times with DELFIA wash solution. Finally, DELFIA enhancement solution (150 µl/well) was added and incubated 5 min at room temperature with slow shaking. The fluorescence was then measured with a time-resolved fluorometer (Wallac).

### Measurement of GDP-Fuc with a1,3-Fucosyltransferase Assay

Yeast cell lysate was used as a fucose donor in a fucosyltransferase reaction converting sLN to sLex (above). The samples were diluted (1:50) with 50 mM MOPS-NaOH buffer (pH 7.5) containing 0.5% Triton X-100 and mixed with other components of enzymatic reaction. For quantitation of GDP-Fuc in samples, defined concentrations of commercial GDP-Fuc diluted with sample buffer were used as standards.

### Measurement of GDP-Fuc with Lectin Inhibition Assay

Lex-polyacrylamide-biotin (0.1 µg/ml in DELFIA assay buffer) was immobilized onto microtitration strips coated with streptavidin. After 30 min incubation and washing, either standards (GDP-Fuc diluted with lysing buffer) or yeast cell lysates (10 µl) were pipetted to wells. Eu-labelled AAL (40 µl, 0.2 µg/ml in DELFIA assay buffer) was then added. After 15 min incubation at room temperature with shaking, the strips were washed six times with DELFIA wash solution. Enhancement solution (150 µl/well) was added and the strips were incubated 5 min at room temperature with slow shaking. The fluorescence was then measured with a time-resolved fluorometer (Wallac).

### Results

Two high-throughput microplate assays were developed for the quantitation of GDP-Fuc. Both assays employ biotinylated carbohydratepolyacrylamide conjugates and time-resolved fluorometric detection. In the first assay (Fig. 2A) GDP-Fuc is used as a fucose donor for FucT enzyme, which transfers fucose to sLN producing sLex. The enzymatic fucosylation reaction proceeds in solution, after which the biotinylated glycoconjugates are specifically captured onto a microtitration plate coated with streptavidin. As constant concentrations of the enzyme and the acceptor are always present in this assay, the concentration of GDP-Fuc in the sample is the limiting factor in the generation of sLex from sLN. The immobilized reaction product sLex is detected with a product-specific primary antibody and europium-labelled secondary antibody. The second assay (Fig. 2B) is based on the binding of europium-labelled fucose-specific lectin AAL to Lex-glycoconjugate immobilized onto microtitration plate wells. GDP-Fuc in the sample binds to the lectin and thus inhibits the binding of the lectin to the immobilized ligand, resulting in a decline in the fluorescence counts.

### Standard Curves for GDP-Fuc

Defined concentrations of commercial GDP-Fuc were used as standards in the assays. In the first assay employing the enzymatic activity of FucT, the fluorescence counts were proportional to the concentration of GDP-Fuc in the range of 10-10 000 nM. The assay was capable of detecting 10 nM GDP-Fuc using enzyme incubation time of 1 h. Background counts were on the level of 10 000 cps and were subtracted from the results. In the second assay, relying on binding of the lectin to either matrix-bound ligand or GDP-Fuc in solution, GDP-Fuc inhibited the binding of AAL to the wells in a dose dependent way. When the binding data was converted to percentages, the inhibition concentration 50% (IC₅₀) was between 50-100 µM. The dynamic range of measurement is narrower than in the enzyme assay, being about 10-500 µM. In the low and high concentration ends of the standard curve the dose-response curve is shallow, which limits the sensitivity of the assay in these concentration areas.

### Assays with Crude Yeast Lysates

The stable recombinant *S.cerevisiae* strain expressing the *E.coli* genes for GMD and GFS needed to convert GDP-Man to GDP-Fuc was tested together with single transformants and controls. As determined with the first, enzyme-based assay, the mock-transformant strain (501-) was unable to synthesize GDP-Fuc, resulting in background fluorescence values. In contrast, the double transformed strain (501+) was able to produce GDP-Fuc from its own GDP-Man pool. The strains expressing either GMD or GFS (single transformants) were unable to produce GDP-Fuc when tested alone. When the lysates of these strains were mixed together, the presence of GDP-Fuc was detected. The same samples were measured also with the second, lectin-binding assay. This assay is less sensitive, but as could be predicted from the standard curves and the results of the enzyme-based assay, the presence of GDP-Fuc could also be detected with this lectin assay. By using the standard curves, concentrations of GDP-Fuc in the cell lysates were calculated. In the lysate of double transformant yeast, the results given by the enzymatic and the lectin assay were 11 µM or 12 µM GDP-Fuc, respectively. Correspondingly, either 17 µM or 25 µM GDP-Fuc was detected in the mixture of the single transformant yeast lysates.

Although the foregoing refers to particularly preferred embodiments, it will be understood that the present invention is not limited to these. It will occur to those ordinarily skilled in the art that various modifications may be made to the disclosed embodiments and that such modifications are intended to be within the scope of the present invention.

All references mentioned herein are incorporated by reference in the disclosure.

## Claims

1. A process for preparing GDP-L-fucose from GDP-D-mannose, wherein the GDP-L-fucose is prepared from inherent GDP-D-mannose by cul-tivating yeast or mold cells, which have been transformed with a DNA sequence coding for GDP-mannose-4,6-dehydratase (GMD) and a DNA sequence coding for GDP-4-keto-6-deoxy-D-mannose-3,5-epimerase/4-reductase (GFS) to functionally express said enzymes, and by recovering the GDP-L-fucose formed from the GDP-D-mannose inherent in said cells.

2. The process of claim 1, wherein the GMD and the GFS expressed are enzymes of *Escherichia coli* or *Helicobacter pylori*.

3. The process of claim 1, comprising transforming yeast cells with a vector comprising said DNA sequences incorporated into a single vector.

4. The process of claim 1, comprising transforming the cells with a vector which encodes a chimeric molecule of GMD and GFS.

5. The process of claim 1, wherein the yeast cell is *Saccharomyces cerevisiae.*

6. The process of claim 1, further comprising preparing fucosylated glycans from the GDP-L-fucose formed.

7. The process of claim 6, wherein the fucosylated glycans are prepared from GDP-L-fucose by recombinant rat or bacterial α-1,3-fucosyltransferase.

8. The process of claim 6, wherein the fucosylated glycans are prepared by *Helicobacter felis α-*1,3*-fucosyltransferase.*

9. The process of claim 1, wherein the cells have been transformed with a DNA sequence encoding *Helicobacter pylori* GDP-mannose-4,6-dehydratase (GMD).

10. The process of claim 1 or 9, wherein the cells have been transformed with a DNA sequence encoding *Helicobacter pylori* GDP-4-keto-6-deoxy-D-mannose-3,5-epimerase/4-reductase (GFS).

11. A chimeric enzyme which is a chimeric molecule of GDP-mannose-4,6-dehydratase (GMD) and GDP-4-keto-6-deoxy-D-mannose-3,5-epimerase/4-reductase (GFS).

12. A vector comprising a DNA sequence encoding a chimeric molecule of GDP-mannose-4,6-dehydratase (GMD) and GDP-4-keto-6-deoxy-D-mannose-3,5-epimerase/4-reductase (GFS).

13. A yeast or mold cell comprising the vector of claim 12.

14. A process for preparing GDP-L-fucose, wherein said compound is prepared from GDP-D-mannose through the *de novo* pathway using at least one of the recombinant enzymes selected from the group consisting of GDP-mannose-4,6-dehydratase (GMD) of *Helicobacter pylori* and GDP-4-keto-6-deoxy-D-mannose-3,5-epimerase/4-reductase (GFS) of *Helicobacter pylori*.

15. The process of claim 14, further comprising preparing fucosylated glycans from the GDP-L-fucose formed.

16. The process of claim 15, wherein the fucosylated glycans are prepared from GDP-L-fucose by recombinant α-1,3-fucosyltransferase, preferably by rat or bacterial α-1,3-fucosyltransferase.

17. An isolated DNA sequence encoding *Helicobacter pylori* GDP-mannose-4,6-dehydratase (GMD).

18. An isolated DNA sequence encoding *Helicobacter pylori* GDP-4-keto-6-deoxy-D-mannose-3,5-epimerase/4-reductase (GFS).

19. An enzyme which is encoded by the DNA sequence of claim 17 or 18.

20. A vector comprising one or more of the DNA sequences of claim 17 or 18.

21. A host cell comprising the vector of claim 20.

22. A process for preparing GDP-L-fucose, wherein said compound is prepared from L-fucose through the salvage pathway using at least one of the recombinant enzymes selected from the group consisting of fuco-1-kinase (FK) and GDP-fucose-pyrophosphorylase (PP).

23. The process of claim 22, comprising transforming host cells with a vector comprising a DNA sequence encoding fuco-1-kinase (FK) and a DNA sequence encoding GDP-fucose-pyrophosphorylase (PP) to obtain transformed host cells that coexpress FK and PP, cultivating said cells, and recovering the GDP-L-fucose formed.

24. The process of claim 1, further comprising preparing fucosylated glycans from the GDP-L-fucose formed.

25. An isolated DNA sequence encoding murine or human fuco-1-kinase (FK).

26. An isolated DNA sequence encoding rat or murine GDP-fucose-pyrophosphorylase (PP).

27. An isolated DNA sequence encoding *Helicobacter felis* or rat α-1,3-fu- cosyltransferase.

28. An enzyme which is encoded by the DNA sequence of any of claims 25 to 27.

29. A vector comprising one or more of the DNA sequences of any of claims 25 to 27.

30. A host cell comprising the vector of claim 29.

31. An assay for the determination of GDP-Fucose or fucosyltransferase said assay comprising employing biotinylated carbohydratepolyacrylamide conjugates, streptavidin and time-resolved fluorometric detection.

32. The assay of claim 31, comprising
incubating a sample suspected to contain GDP-fucose or fucosyltransferase, respectively, with a fucosyletransferase or with GDP-fucose, respectively, and a sLN-polyacrylamide-biotin conjugate to form a biotinylated fucosylated glycoconjugate (sLex conjugate)
contacting the reaction mixture of the previous step with immobilized streptavidin to immobilize the biotinylated sLex conjugate,
reacting the biotinylated sLex conjugate with a primary anti-sLexantibody, and then with a secondary europium-labelled antibody that recognizes the primary antibody, and
detecting any time-resolved fluoresence as a measure of GDP-fucose or fucosyltransferase, respectively, in the sample.

33. The assay of claim 31, comprising
immobilizing a biotinylated Lex-polyacrylamide conjugate onto a carrier coated with streptavidin,
adding a sample suspected to contain GDP-fucose, and europiumlabelled fucose-specific lectin ML, incubating and washing, and
detecting any time-resolved fluorescence, whereby a decrease in the fluorescence indicates the amount of GDP-Fuc present in the sample.

34. Test kit comprising reagents needed for performing the assay of claim 31.
